# EUROPÄISCHE PATENTSCHRIFT

(11) **EP 4 034 208 B1**
(45) Veröffentlichungstag und Bekanntmachung des Hinweises auf die Patenterteilung: **13.08.2025**
(21) Anmeldenummer: 20797548.3
(22) Anmeldetag: 19.09.2020
(51) Int. Cl.: A61M 16/04, A61M 25/10

(54) **FLUSSOPTIMIERTE ZULEITUNG ZU EINEM ORGAN-SYNCHRON, DYNAMISCH DICHTENDEN BALLONELEMENT**
FLOW-OPTIMISED SUPPLY TO A BALLOON ELEMENT THAT SEALS DYNAMICALLY AND IN SYNC WITH ORGANS
CONDUITE D'ALIMENTATION À FLUX OPTIMISÉ VERS UN ÉLÉMENT DE TYPE BALLONNET À ÉTANCHÉIFICATION DYNAMIQUE, SYNCHRONE D'UN ORGANE

(30) Priorität: 24.09.2019 DE 102019006680
(43) Veröffentlichungstag der Anmeldung: 03.08.2022
(73) Patentinhaber: Advanced Medical Balloons GmbH, 68753 Waghäusel (DE)
(72) Erfinder: GÖBEL, Fred, 67346 Speyer (DE)
(74) Vertreter: Küchler, Stefan
(86) Internationale Anmeldenummer: PCT/IB2020/058935
(87) Internationale Veröffentlichungsnummer: WO 2021/059183

(56) Entgegenhaltungen:
- WO-A1-2013/139986
- WO-A1-2014/090680
- WO-A1-2016/087930
- WO-A1-2017/085540
- WO-A1-99/45991
- DE-A1- 3 028 568
- US-A- 3 731 692
- US-A- 3 794 043
- US-A- 4 159 722
- US-A- 4 856 510
- US-A- 5 235 973
- US-A- 5 247 927
- US-A1- 2010 326 446
- US-A1- 2019 262 562
- US-B1- 6 647 984

## Beschreibung

Die Erfindung richtet sich auf Vorrichtungen zur organsynchronen, dynamischen Okklusion, Dichtung und/oder Tamponade eines Hohlorgans mittels eines ballonartigen Elementes, insbesondere auf die dynamisch adaptierende, aspirationspräventive Sekretdichtung der intubierten Trachea bei eigenständig atmenden Patienten sowie bei Patienten, die in einem unterstützenden Spontanatmungsmodus maschinell beatmet werden. Insbesondere richtet sich die Erfindung auf eine Vorrichtung zur dynamisch adaptierenden Dichtung eines Organs oder einer Körperhöhle, beispielsweise der Luftröhre (Trachea) eines intubierten und beatmeten Patienten, insbesondere durch ein dichtendes Ballonelement, wobei im beispielhaften Falle der dynamischen Dichtung der Trachea, ein vorzugsweise im Durchmesser residual ausgeformter, also den trachealen Durchmesser überschreitender, ballonartiger Folienkörper der inneren Wandung der Trachea mit einem möglichst konstant wirkenden Druck in dichtender Weise anliegt; dabei sollen Schwankungen des Ballonvolumens, die durch atemmechanisch bedingte Schwankungen des intra-thorakalen Drucks verursacht werden, mit geringstmöglicher zeitlicher Latenz durch Zufuhr von Volumen aus einem extrakorporalen Reservoir oder einer extrakorporalen Quelle kompensiert werden, damit die tracheale Sekretdichtung des Ballons durchgängig erhalten bleibt.

Im Besonderen richtet sich die Erfindung auf eine Vorrichtung für die organsynchron volumenkompensierende Dichtung eines Hohlorgans oder eines anatomische Raumes mit einem vorgegebenen anatomischen Maß, umfassend (i) einen intrakorporalen, ballonartigen Folienkörper mit dichtenden Flächen zur Anlage an der Wandung des jeweiligen Hohlorgans oder Raumes, während der folienartige Ballonkörper selbst mit einem Füllmedium unter einem Solldruck von höchstens 50 mbar befüllt ist, vorzugsweise unter einem Solldruck von höchstens 40 mbar, insbesondere unter einem Solldruck von höchstens 30 mbar, (ii) einen Tubus oder sonstigen Schaft, welchem der ballonartige Formkörper aufsitzt, (iii) eine extrakorporale Regeleinrichtung mit einem Volumenreservoir und/oder einer Druckquelle für das Füllmedium, sowie (iv) eine Strömungsverbindung zwischen dem intrakorporalen ballonartigen Folienkörper und der extrakorporalen Regeleinrichtung, die zumindest bereichsweise in oder entlang des Tubus oder sonstigem Schaft verläuft, wobei in der Strömungsverbindung extrakorporal ein Konnektor vorgesehen ist.

Ein grundsätzliches Problem beim organverträglichen, effizient dichtenden Verschluss oder bei der raumfüllenden Tamponade von Organen oder Kavitäten mit einem von extrakorporal befüllten, ballonartigen Element, ist eine fortwährende, mehr oder weniger stark ausgeprägte Motilität des Organs selbst. Muskulösbindegewebig begrenzte Organe oder Körperhöhlen weisen häufig eine charakteristische Bewegungsdynamik auf oder sind in typischer Weise der Dynamik angrenzender Organe oder Strukturen ausgesetzt. Derartig eigenmotile oder korrespondierend motile Organe erfordern für einen kontinuierlich wirkenden Verschluss des Organlumens einen besonderen, rasch auf Schwankungen des Organdurchmessers und/oder auf Änderungen des Tonus der Organwandung reagierenden und diese kompensierenden Regelmechanismus. Der Mechanismus muss möglichst zeitsynchron zur jeweiligen Durchmesser- oder Tonusveränderung im Organ wirken und durch eine optimal rasche Zu- oder auch Ableitung von Füllmedium in den Ballon dessen verschließende oder dichtende Eigenschaften aufrechterhalten.

Das Problem einer sich dynamisch adaptierenden Dichtung eines Hohlorgans kann am Beispiel der menschlichen Luftröhre veranschaulicht werden. Die Luftröhre (Trachea) ist eine aus knorpeligen, bindegewebigen und muskulös-bindegewebigen Anteilen aufgebaute, rohrartige Struktur. Sie reicht vom unteren Ende des Kehlkopfes bis zur Verzweigung in die beiden Hauptbronchien. Die vorderen und die seitlichen Anteile der Luftröhre sind dabei durch spangenartige, in etwa hufeisenförmige Strukturen stabilisiert, die wiederum durch bindegewebige Lagen in Längsrichtung miteinander verbunden sind. Auf der rückwandigen Seite wird das Luftröhrenlumen durch die sogenannte Pars membranacea abgeschlossen, die aus durchgängig muskulös-bindegewebigen Lagen, ohne darin integrierte, versteifende Elemente, besteht. Ihr liegt dorsal wiederum die muskulös-bindegewebige Speiseröhre (Ösophagus) an.

Das obere Drittel der Trachea befindet sich in der Regel außerhalb des Brustkorbes (Thorax), während die beiden unteren Drittel innerhalb der von Brustkorb und Zwerchfell abgegrenzten Thoraxhöhle liegen. Der untere, thorakale Anteil der Trachea ist so, in besonderer Weise, den Druckschwankungen in der Brusthöhle ausgesetzt, die sich im Rahmen der "thorakalen Atemarbeit" eines spontan, nicht unterstützt atmenden oder auch eines maschinell assistiert beatmeten Patienten einstellen.

Bei der Einatmung (Inspiration) des Patienten wird das thorakale Volumen durch die Hebung der Rippen und simultane Senkung des Zwerchfells vergrößert, wodurch der im Thorax herrschende, intra-thorakale Druck abfällt. Der Druckabfall im Thorax führt zum Einströmen von Atemluft in die dem Thorax folgende, sich elastisch aufdehnende Lunge.

Der mit der thorakalen Volumenvergrößerung einhergehende Abfall des intra-thorakalen Drucks verursacht in dichtend und/oder tamponierenden Ballonelementen, die innerhalb der thorakalen Höhle positioniert, und dort mit einem bestimmten Fülldruck beaufschlagt sind, zur thorakalen Atmung des Patienten korrespondierende Druckschwankungen. Dies wird zum Beispiel bei den dichtenden Ballonelementen (Cuff-Manschetten) von Trachealtuben und Tracheostomiekanülen beobachtet, die die tiefen Luftwege gegen einströmende Sekrete des Rachens dichten, sowie eine positive Druckbeatmung der Patientenlunge (positive pressure ventilation - PPV) ermöglichen.

Die zyklischen, durch die vom Patienten bei der thorakalen Atmung erzeugten, intra-thorakalen Druckschwankungen können den dichtungswirksamen Druck in den Ballons von Beatmungskathetern in Bereiche bewegen, in denen eine suffiziente Dichtung gegen die Sekrete des Rachens und des Verdauungstraktes nicht mehr gewährleistet ist. Der Cuffdruck kann im Rahmen der thorakalen Eigenatmung des Patienten unter Umständen auch subatmosphärische Werte annehmen, die dem jeweils wirkenden thorakalen Druck nahezu entsprechen. Siehe hierzu Badenhorst CH, Changes in tracheal cuff pressure in respiratory support, CritCareMed, 1987; 15/4: 300-302.

Während die Dichtungsleistung herkömmlicher Trachealtuben-Cuffs aus PVC eng mit dem aktuell im Cuff herrschenden Fülldruck korreliert und bereits im Druckbereich von 30 mbar bis auf 15 mbar absteigend einen hierzu korrelierenden Abfall der Dichtungsleistung zur Folge hat, verfügen besonders dünnwandig hergestellte Trachealtuben-Cuffs aus Polyurethan über eine deutlich stabilere Dichtungseffizienz, wenn der im Cuff herrschende Fülldruck von 30 auf 15 mbar absinkt, siehe Bassi GL, CritCareMed, 2013; 41: 518-526.

Von wesentlich größerer Bedeutung für die Sekretdichtungseffizienz trachealer Cuffs ist jedoch der Fülldruckbereich von 15 bis 5 mbar, der quasi von Atemhub zu Atemhub, bereits bei moderat forcierter Atemarbeit erreicht werden kann. Dichtungsoptimierte, mikrodünnwandige PUR-Cuffs bieten zwar auch bei ca. 10 mbar Fülldruck gute Dichtungsleistungen, können bei Druckabfällen auf unter 10 mbar bzw. auf subatmosphärische, intra-thorakale Druckwerte jedoch nicht gegen einströmendes subglottisches Sekret schützen.

Eine der Eigenatmung des Patienten zeitsynchron folgende, über ausreichend weite Fülldruckbereiche dichtungseffizient und atraumatisch wirkende, kostengünstig herstellbare Verschlusstechnik der Luftröhre durch einen cuff-artigen Dichtungsballon ist bislang nicht verfügbar. Zwar sind im Stand der Technik verschiedenste Ausführungen extrakorporaler, fülldruckregulierender Vorrichtungen für tracheale Beatmungskatheter beschrieben, eine tatsächlich zeitsynchrone, also von Hub zu Hub wirksame Anpassung des Dichtungsdrucks an alternierende thorakale Drucke, wie sie bei der Eigenatmung des Patienten herrschen, gibt es im Stand der Technik derzeit jedoch nicht.

Bei konventionellen Beatmungskathetern erfolgt die Befüllung des tracheal dichtenden Cuffs in der Regel durch kleinlumige, in die Wandung des Katheterschaftes extrudierte, kanalartige Zuleitungen. Die niedrigen Querschnitte der Befüll-Leitungen von ca. 0,5 mm gewährleisten in der Regel keinen ausreichend großen Volumenstrom des den Cuff mit Druck beaufschlagenden Füllmediums, um die tracheale Dichtung des Cuffs über einen vollständigen Atmungszyklus des Patienten hinweg aufrechtzuerhalten. Selbst technisch aufwendige, elektronisch gesteuerte Regelmechanismen, wie beispielsweise das Gerät CDR 2000 der Firma Logomed GmbH (nicht mehr im Handel), bieten, durch die traditionelle Kleinlumigkeit der Zuleitung zwischen dem dichtenden Ballonelement und dem außerhalb des Körpers platzierten Regler bedingt, ebenfalls nur eine unzureichende Dichtungseffizienz.

Die vorliegende Erfindung geht aus von einer Anordnung, wobei in der Strömungsverbindung des Füllschlauchs ein extrakorporaler Konnektor vorgesehen ist, über welchen ein Reservoir oder eine Quelle an den Füllschlauch anschließbar ist.

Demgegenüber richtet sich die DE 30 28 568 A1 auf einen Endotrachealtubus zur Beatmung eines Patienten. Ein extrakorporaler Konnektor ist dabei jedoch nicht offenbart, so dass bei diesem Endotrachealtubus ein lösbarer Anschluss eines Reservoirs oder einer Quelle an den Füllschlauch nicht vorgesehen ist.

In ähnlicher Form offenbart die US 4,856,510 zwar ebenfalls einen Trachealtubus, jedoch ebenfalls ohne einen extrakorporalen Konnektor, so dass auch hier kein lösbarer Anschluss eines Reservoirs oder einer Quelle oder einer sonstigen Regeleinrichtung an dem Füllschlauch möglich ist.

Entsprechendes gilt für die US 5,247,927. Diese offenbart zwar einen Trachealtubus mit einem Cuff bzw. Ballon, jedoch ohne jeglichen Konnektor, so dass auch dort weder ein Reservoir noch eine Quelle noch eine sonstige Regeleinrichtung lösbar an den Füllschlauch angeschlossen werden kann.

Bei der WO 2014/090680 A1 ist ein derartiger Konnektor allenfalls in mit einem Dämpfungsventil integrierter Form vorhanden. Im Rahmen dieses Dämpfungsventils sind jedoch keinerlei Maßnahmen getroffen, um eine tubulente Strömung in dem betreffenden Bereich zu vermeiden.

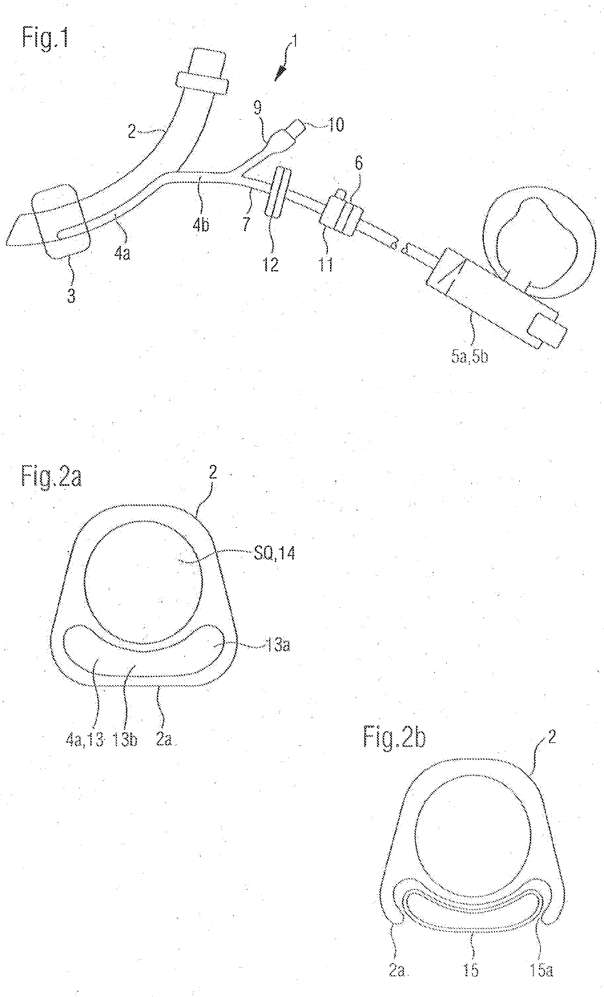

Die US 4,159,722 offenbart einen Druckregler für den Cuff bzw. Ballon eines Endotrachealtubus. in der dortigen Fig.2 erkennt man jedoch, dass der Ballon ohne Einfaltungen an der Wandung des betreffenden Hohlorgans anliegt, also nicht residual bemessen ist. Ferner sind dort im Bereich eines Schlauchs zwischen dem Ballon an dem Tubus einerseits und der Regeleinrichtung selbst keine Maßnahmen getroffen, um eine turbulente Strömung zu vermeiden. Insbesondere gibt es im Bereich dieser Steckverbindungen jeweils Kanten und Ausstülpungen, welche einer laminaren Strömung im Wege stehen und damit einen schnellen Druckausgleich innerhalb des Cuffs bzw. Ballons im Wege stehen.

Dasselbe gilt für die US 6,647,984 B1, die eine Vorrichtung zur Steuerung des Druckes innerhalb des Cuffs oder Ballons eines Endotrachealtubus betrifft. Auch dort sind zwischen dem Ballonkörper und der umgebenden Trachea keinerlei Einfaltungen erkennbar, so dass es an einer residualen Bemessung des Cuffs mangelt und demnach innerhalb desselben ein erhöhter Druck herrscht, welcher einen schnellen Druckausgleich nicht erfordert.

Dasselbe ist bei US 3 794 043 in Fig. 1 zu sehen; auch dort weist der entfaltete Ballonkörper innerhalb des Hohlorgans keine Einfaltungen auf, was ein untrügliches Indiz dafür ist, dass innerhalb des Ballons ein erhöhter Druck herrscht und somit ein rascher Druckausgleich nicht notwendig ist.

Die US 5,235,973 beschreibt eine relativ aufwendige, elektronisch gesteuerte Regeltechnik, die auf eine besonders rasche Druckregulation in einem dichtenden Katheterballonelement ausgerichtet ist. Sie beschreibt u.a. bevorzugte Querschnitte der Zuleitung, welche den Katheterballon mit der druckregelnden Vorrichtung verbindet. Die genannten Durchmesser liegen im Bereich von ca. 2 bis 3 mm. Zur Reduktion des Flusswiderstandes innerhalb der Zuleitung zum tracheal dichtenden Cuff, der im Wesentlichen vom Durchmesser des in den Katheterschaft integrierten bzw. extrudierten, zuleitenden Lumens bestimmt wird, schlagen die WO 2016/087930 A1 und WO 2017/085540 A1 entsprechend dimensionierte Zuleitungsquerschnitte vor.

Es hat sich jedoch herausgestellt, dass die Zuleitungsquerschnitte alleine keine Gewähr für einen möglichst schnellen Druckausgleich in einem intrakorporalen Dichtungsballon darstellen, sondern dass die gesamte bauliche Ausgestaltung des Strömungskanals zwischen einem intrakorporalen Dichtungsballons einerseits und einer extrakorporalen Regeleinrichtung andererseits Einfluss auf die erreichbare Strömungsgeschwindigkeit und damit auf das kurzzeitig verschiebbare Fülldruckvolumen hat. Weder die US 5,235,973 noch die WO 2016/087930 A1 noch die WO 2017/085540 A1 gehen auf diese Problematik ein.

Aus diesen Nachteilen des beschriebenen Standes der Technik resultiert das die Erfindung initiierende Problem, den Strömungskanal zwischen einem intrakorporalen Dichtungsballon einerseits und einer extrakorporalen Regeleinrichtung andererseits derart auszugestalten, dass zum Einen die extrakorporale Regeleinrichtung an den Füllschlauch anschließbar und von jenem wieder lösbar ist, wobei zum Anderen ein möglichst schneller Druckausgleich in einem intrakorporalen Dichtungsballon erreichbar sein soll.

Die Lösung dieses Problems gelingt im Rahmen einer Vorrichtung zur organsynchron volumenkompensierenden Dichtung eines Hohlorgans oder eines anatomische Raumes, umfassend (i) einen intrakorporalen, auf ein residuales Maß, also das anatomische Maß des Organs oder des jeweiligen Raumes überschreitend, ausgeformten, ballonartigen Folienkörper mit dichtenden Flächen, welche der Wandung des jeweiligen Hohlorgans oder Raumes bei spannungslos ungedehnten ballonartigen Folienkörper zumindest bereichsweise unter Ausbildung von Einfaltungen anliegen, während der folienartige Ballonkörper selbst mit einem Füllmedium unter einem Solldruck von höchstens 50 mbar befüllt ist, vorzugsweise unter einem Solldruck von höchstens 40 mbar, insbesondere unter einem Solldruck von höchstens 30 mbar, (ii) einen Tubus oder sonstigen Schaft, welchem der ballonartige Formkörper aufsitzt, (iii) eine extrakorporale Regeleinrichtung mit einem Volumenreservoir und/oder einer Druckquelle für das Füllmedium, sowie (iv) eine Strömungsverbindung zwischen dem intrakorporalen ballonartigen Folienkörper und der extrakorporalen Regeleinrichtung, die zumindest bereichsweise in oder entlang des Tubus oder sonstigem Schaft verläuft, wobei in der Strömungsverbindung extrakorporal ein Konnektor vorgesehen ist, dadurch, dass
a) der ballonartige Formkörper auf ein residuales, das anatomische Maß des Organs oder des jeweiligen Raumes überschreitendes Maß ausgeformt ist, derart, dass die dichtenden Flächen des ballonartigen Folienkörpers der Wandung des jeweiligen Hohlorgans oder Raumes bei spannungslos ungedehtem ballonartigen Folienkörper zumindest bereichsweise unter Ausbildung von Einfaltungen anliegen, und dass
b) die Strömungsverbindung
   b1) zwischen dem intrakorporalen ballonartigen Folienkörper und der extrakorporalen Regeleinrichtung in dem Bereich ihres Verlaufs in oder entlang des Tubus oder sonstigen Schaftes einschließlich eines Übergangsbereichs von dem Tubus oder sonstigen Schaft zu einem davon losgelösten Verlauf frei von rechtwinkligen Umlenkungen ist, so dass sich dort eine laminare Strömung ausbilden kann und innerhalb einer Latenzzeit von 200 ms oder weniger, beispielsweise von 100 ms oder weniger, vorzugsweise von 50 ms oder weniger, insbesondere von 25 ms oder weniger, die in dem ballonartigen Folienkörper benötigte zusätzliche Füllmenge des Füllmediums ergänzt werden kann, um Schwankungen des Ballonfülldrucks und/oder des Ballonvolumens und/oder der am ballonartigen Folienkörper anlastenden Drucke und Kräfte zu kompensieren, so dass die Dichtung oder die raumfüllende Tamponade des Hohlorgans oder Raums unter dynamisch alternierenden Schwankungen des Ballonfülldrucks bei einem Druckabfall in dem ballonartigen Folienkörper um 30 mbar erhalten bleibt, und
   b2) im Bereich des Übergangs zwischen verschiedenen Komponenten, insbesondere im Bereich des Konnektors, frei von Knicken ist und frei von Kanten und frei von Abstufungen und frei von Spalten und frei von Graten und frei von sonstigen abrupten Erhebungen oder Vertiefungen, um die laminare Strömung nicht zu beeinträchtigen.

Die Erfindung berücksichtigt damit das besondere Erfordernis der Vermeidung flussreduzierender bzw. den optimal raschen Druckausgleich zwischen Dichtungsballon und Regler behindernder Übergänge und Hindernisse innerhalb der zum Ballon führenden Zuleitung.

Der Erfinder hat erkannt, dass insbesondere die spezifische bauliche Ausführung des Übergangs der schaftintegrierten Zuleitung in eine sich an den Schaft anschließende, schlauchförmige Zuleitung, oder auch die spezifische bauliche Gestaltung im Bereich des Austritts von Füllmedium aus der schaftintegrierten Zuleitung in die Cuff-Manschette, den Volumenfluss reduzieren und dichtungsrelevant verzögern können. Vor allem beim Druckausgleich aus einem isobar zum dichtenden Cuff mit Druck beaufschlagten, extrakorporalen Reservoir sind strömungsbehindernden Übergänge, wegen der in der Regel kleinen, den Volumenfluss antreibenden Druckdifferenzen problematisch. Die resultierende Verzögerung beim Druckausgleich zwischen den beiden miteinander kommunizierenden Kompartimenten, Reservoir und Cuff, schließen eine kontinuierlich verlässliche Dichtung aus. Die sich zyklisch einstellenden Druckgradienten bewegen sich im Bereich weniger Millibar, typischerweise in einem Druckbereich von etwa 5 bis 30 mbar. Der den Volumenfluss vom Reservoir oder der Volumenquelle zum Cuff treibende Druckgradient setzt deshalb die im Folgenden beschriebene, in besonderer Weise fluss-optimierte Gestaltung sämtlicher Komponenten sowie sämtlicher Übergänge zwischen den Komponenten voraus, aus denen die Zuleitung zwischen Reservoir und Cuff in ihrer Gesamtheit aufgebaut ist.

Es hat sich als günstig erwiesen, dass der Tubus oder der sonstige Schaft aus einem derart begrenzt flexiblen Werkstoff besteht, dass er biegsam ist, aber nicht knickbar. Damit kann sich der Tubus oder der sonstige Schaft einerseits der Anatomie eines Patienten optimal anpassen, ohne dass seine Funktionsweise beeinträchtigt werden könnte. Insbesondere könnte ein Knick eine Volumenverschiebung erheblich verzögern oder gar unmöglich machen.

Da beispielsweise Stufen, Grate, unebene Oberflächen oder sonstige nicht geglättete Strukturen den laminaren Fluss des Füllmediums in turbulenter Weise unterbrechen, Sieht die Erfindung vor, dass die Strömungsverbindung im Bereich des Tubus oder des sonstigen Schaftes und/oder im Bereich eines Übergangs zwischen verschiedenen Komponenten frei von Knicken ist und/oder frei von Kanten und/oder frei von Abstufungen und/oder frei von Spalten und/oder frei von Graten und/oder frei von sonsten abrupten Erhebungen oder Vertiefungen, um die laminare Strömung nicht zu beeinträchtigen.

Die Erfindung erfährt eine vorteilhafte Weiterbildung dadurch, dass die Strömungsverbindung im Bereich des Tubus oder des sonstigen Schaftes frei von Biegungen ist, deren Biegeradius in Längsrichtung der Strömung kleiner ist als 0,5 cm, beispielsweise kleiner als 1 cm, vorzugsweise kleiner als 2 cm, insbesondere kleiner als 5 cm. Wie Knicke, so können sich auch enge Richtungsänderungen des Strömungskanals negativ auf die erreichbare Strömungsgeschwindigkeit auswirken, insbesondere weil dadurch die Tendenz zur Ausbildung von Wirbeln gesteigert würde, und aus diesem Grund sollte auf derart enge Biegungen verzichtet werden.

Es liegt im Rahmen der Erfindung, dass sich die Querschnittsfläche der Strömungsverbindung von dem Bereich des Tubus oder des sonstigen Schaftes ausgehend bis hin zu der extrakorporalen Regeleinrichtung nicht verringert. Jeglicher Rückstau innerhalb des Strömungskanals reduziert die erzielbare Strömungsgeschwindigkeit und sollte daher vermieden werden.

Besondere Vorteile ergeben sich dadurch, dass sich die Querschnittsfläche der Strömungsverbindung in dem Übergangsbereich von dem Tubus oder dem sonstigen Schaft zu einem davon losgelösten Verlauf vergrößert. Solchenfalls kann sich ein ggf. höherer Druck innerhalb der Regeleinrichtung nahezu uneingeschränkt ausbreiten bis zu dem Übergangsbereich in den Tubus, so dass dort eine maximale Druckdifferenz zur Verfügung steht, um innerhalb des dortigen, begrenzten Strömungsquerschnitts eine maximale volumenverschiebung zu treiben.

Es hat sich bewährt, dass der Querschnitt der Strömungsverbindung in oder entlang des Tubus oder des sonstigen Schaftes eine bogenförmige Gestalt aufweist, die sich vorzugsweise einem funktionellen Lumen innerhalb des Tubus oder des sonstigen Schaftes etwa tangential anschmiegt oder jenes koaxial umgibt. Einerseits ist es dank einer solchen Ausgestaltung möglich, einen relativ kreisrunden Gesamtquerschnitt des Tubus beizubehalten, entsprechend der meistens ebenfalls querschnittlich etwa kreisförmigen Lumina des menschlichen Körpers; andererseits hat eine bogenförmig längliche Querschnittsform der Strömungsverbindung den Vorteil, dass eingedrungene Flüssigkeit den Strömungskanal nicht auf dessen gesamter Querschnittsfläche verschließen kann, was mit einer erheblichen Beeinträchtigung des Druckausgleiches einherginge.

Nur bei möglichst großlumigen und tendenziell kurzen, zuleitenden Komponenten kann, im Verbund mit verwirbelungsminimierenden Übergängen zwischen den Komponenten, ein optimal verzögerungsfreier, möglichst laminarer Fluss des dichtenden Mediums aus einem Volumenreservoir oder aus einer elektronisch/elektro-mechanisch regulierten Volumenquelle zum dichtenden Ballon hergestellt, und somit eine suffizient rasche, dynamische Dichtung der Trachea, von Atemhub zu Atemhub, aufrechterhalten werden.

Die Erfindung erlaubt auch eine Realisierung dergestalt, dass die Strömungsverbindung im Bereich des Tubus oder sonstigen Schaftes als jenem an dessen Außenseite anfügbare Leitung oder Schlauchleitung ausgebildet ist, so dass sich die Anzahl von Übergängen zwischen verschiedenen Komponenten des Strömungskanals - wo Wirbel ausgelöst werden könnten - reduzieren lässt.

Zur Aufnahme einer derartig anfügbaren Leitung oder Schlauchleitung kann in der Außenseite des Tubus oder des sonstigen Schaftes eine muldenförmige Rinne oder Vertiefung eingeformt sein. Dies hätte den Vorteil, dass der für die Dichtung des Ballons wichtige Strömungskanal im Bereich des Tubus nicht exponiert ist und also gequetscht werden könnte, sondern in einer Vertiefung des Tubus geschützt angeordnet ist.

Im Rahmen einer bevorzugten Weiterbildung der Erfindung kann die muldenförmige Rinne oder Vertiefung seitliche Hinterschneidungen aufweisen, so dass eine dort eindrückbare oder einsteckbare Leitung oder Schlauchleitung festgehalten wird und sich nicht selbsttätig lösen kann, wodurch die Handhabung erleichtert wird.

Außerdem besteht die Möglichkeit, dass die dem Tubus oder sonstigen Schaft an dessen Außenseite anfügbare Leitung oder Schlauchleitung derart präformiert ist, dass sie die muldenförmige Rinne oder Vertiefung ausfüllt und dabei die angrenzenden Außenkonturen des Tubus oder sonstigen Schaftes konturerhaltend ergänzt. Solchenfalls kann der betreffende Tubus oder Schaft auch im Bereich der Schlauchleitung atraumatisch in einem Lumen des menschlichen Körpers platziert werden.

Die Erfindung erlaubt eine Weiterbildung dahingehend, dass in dem Übergangsbereich der Strömungsverbindung von einem in den Tubus oder in einen sonstigen Schaft integrierten oder eingeformten Strömungskanalabschnitt zu einem davon losgelösten Verlauf des Strömungskanals eine Komponente mit einem rampen- oder bogenförmigen Verlauf vorgesehen, insbesondere eingefügt ist. Die Aufgabe einer solchen Komponente besteht darin, die Strömung des Füllmediums, bspw. Luft, in diesem Übergangsbereich sanft und verwirbelungsfrei umzuleiten, um die laminare Strömung nicht zu beeinträchtigen.

Diesem Erfindungsgedanken weiter folgend kann vorgesehen sein, dass eine lösbare Komponente mittels eines rückwärtigen, an einer der Rampe abgewandten Seite angeordneten, vorzugsweise dornartigen Fortsatz in eine mit dem in den Tubus oder in einen sonstigen Schaft integrierten oder eingeformten Strömungskanalabschnitt fluchtende Vertiefung eingesteckt ist. Dadurch kann gewährleistet werden, dass eine solche Komponente stets optimal ausgerichtet ist und nicht etwa durch eine Schrägstellung od. dgl. Spalten oder Kanten entstehen, welche die Strömung beeinträchtigen könnten. Natürlich kann eine solche Komponente auch zusätzlich mittels Klebstoff lagemäßig fixiert werden.

Ferner kann vorgesehen sein, dass in dem Übergangsbereich der Strömungsverbindung von einem in den Tubus oder in einen sonstigen Schaft integrierten oder eingeformten Strömungskanalabschnitt zu einem davon losgelösten Verlauf des Strömungskanals eine Komponente mit einer rohrförmigen Gestalt und einem sanft gebogenen Verlauf aus einem knickstabilen Material eingefügt ist. Auch einer solchen Komponente obliegt es primär, für eine sanfte Änderung der Strömungsrichtung in dem Übergangsbereich zu sorgen und dort der Entstehung einer turbulenten Strömung entgegenzuwirken.

Wenn der Außenquerschnitt der Komponente von rohrförmiger Gestalt größer ist als der Innenquerschnitt des in den Tubus oder in einen sonstigen Schaft integrierten oder eingeformten Strömungskanalabschnitts, so ist er dort unter lokaler Aufdehnung des Strömungskanalabschnitts reibschlüssig festlegbar und erfährt dadurch eine führende Ausrichtung in Längsrichtung des in den Tubus oder sonstigen Schaft eingeformten Strömungskanal.

Eine bevorzugte Ausführungform der Erfindung zeichnet sich dadurch aus, dass in dem Übergangsbereich der Strömungsverbindung von einem in den Tubus oder in einen sonstigen Schaft integrierten oder eingeformten Strömungskanalabschnitt zu einem davon losgelösten Verlauf des Strömungskanals eine Komponente aus einem dünnwandigen, sich der Mündung des Strömungskanals in dem Tubus oder sonstigen Schaft anschmiegenden Material eingefügt ist. Eine Aufgabe einer solchen Komponente besteht darin, im Bereich des Einschnittes in den Tubus oder sonstigen Schaft, wodurch jener strukturell bereichsweise geschwächt wird, als Verstärkung zu dienen, um dort die Entstehung eines unerwünschten Knickes zu vermeiden.

Eine besondere Ausgestaltung erfährt die Erfindung dadurch, dass in dem Übergangsbereich der Strömungsverbindung von einem in den Tubus oder in einen sonstigen Schaft integrierten oder eingeformten Strömungskanalabschnitt zu einem davon losgelösten Verlauf des Strömungskanals eine haubenförmige Komponente mit lateralen, sattelartig flächigen Ausläufern an- oder eingefügt ist, wobei sich die Ausläufer vorzugsweise mit dem davon abgedeckten Tubenschaft stabilisierend verbinden lassen, beispielsweise adhäsiv. Auch diese lateralen, sattelartig flächigen Ausläufer dienen sowohl der Ausrichtung gegenüber dem Tuben- oder Schaftmantel als auch der Festlegung an eben diesem Tuben- oder Schaftmantel.

Im Rahmen einer besonders bevorzugten Ausführungsform der Erfindung ist eine Komponente mit einem rampen- oder bogenförmigen Verlauf von einer haubenförmigen Komponente abgedeckt. Gemäß der Erfindung können mehrere solche Komponenten miteinander kombiniert werden, insbesondere je eine in den tubusinternen Strömungskanal oder dessen Verlängerung eingesteckte Übergangskomponente und eine an der Außenseite des Tubus oder sonstigen Schaftes anliegende Übergangskomponente.

Vergleichbar zu allen vorab beschriebenen Übergangselementen ausgestaltete Geometrien können auch an dem Übergangsbereich zwischen dem distalen Ende des tubus- oder schaftintegrierten Strömungskanals und dem dort auf dem Tubus oder Schaft applizierten Dichtungsballon vorgesehen sein.

Im Allgemeinen kann eine in dem Übergangsbereich der Strömungsverbindung von einem in den Tubus oder in einen sonstigen Schaft integrierten oder eingeformten Strömungskanalabschnitt zu einem davon losgelösten Verlauf des Strömungskanals angeordnete Komponente im Bereich ihres proximalen Endes mit einer Muffe zum Auf- oder Einstecken eines Schlauchs versehen sein. Falls dort der Schlauch eine radiale Erweiterung an seiner Innenseite aufweist, können Abstufungen in diesem Bereich vermieden werden.

In den Segmenten bzw. Komponenten der Zuleitung, die außerhalb des Körpers liegen, und deren bauliche Gestaltung oder Dimensionierung nicht durch anatomische Vorgaben begrenzt wird, lässt sich das Design der Zuleitung in der Regel den Erfordernissen der Flussmaximierung anpassen. Insbesondere lassen sich dort größere Zuleitungsleitungsquerschnitte realisieren. Beim beschriebenen Beispiel eines Trachealtubus oder einer Tracheostomiekanüle bestehen anatomisch bedingte Limitierungen in den Zuleitungen zum tracheal dichtenden Cuff vor allem im Bereich der den Cuff tragenden schlauch- bzw. rohrartigen Schaftkomponente. Insbesondere die Ebene der Stimmlippen (Glottis) ist für die Bemaßung des Tubenschaftes limitierend. Sie ist die engste Stelle in den Luftwegen und gibt die möglichen Dimensionen der jeweilig möglichen äußeren Tubendurchmesser, und damit im Wesentlichen auch der Durchmesser der inneren, in den Schaft hineinextrudierten Tubenlumina vor. Die Querschnittfläche des für die Beatmung der Lunge verwendete Tubenlumens ist wegen des möglichst klein zuhaltenden Atemwiderstandes bei der Beatmung grundsätzlich zu maximieren. In der intubierten Stimmlippenebene verbleibt in der Regel im dorsalen Bereich ein gewisser Restraum zum Tubenschaft, wobei sich sie Stimmlippen, in der Art eines Vorhangs von ventral nach dorsal zeltartig, triangulär aufspannen hin aufschlagen und am Boden des zeltartigen Vorhangs bzw. der Glottis einen zwickelförmigen Bereich zwischen der äußeren Wandung des Tubenschaftes und dem Glottisboden freigeben. Dieser kann für zusätzliche und/oder vergrößerte schaftintegrierte Lumina, im Sinne einer fluss-optimierten Zuleitung oder auch Ableitung eines Füllmediums zum Cuff genutzt werden kann. Die Erfindung schlägt hierzu u.a. ein annähernd hantelförmiges Profil einer Zuleitung in der dorsal positionierten Rückwand des Tubenschaftes vor, dessen besondere, laterale Erweiterungen des hantelförmigen Querschnitts diesen Restraum ausfüllen bzw. nutzen.

Die Länge der schaftintegrierten Zuleitung zum dichtenden Ballonelement ist in der idealen Bauform des jeweiligen Tubus oder der jeweiligen Kanüle möglichst kurz ausgeführt, und reicht von der Öffnung der Zuleitung in den Cuff bis zu einem unmittelbar oberhalb, beispielsweise 2 bis 3 cm von der Glottis beabstandeten Punkt. Bereits im supra-glottischen Hypopharynx kann ein, relativ zum schaftintegrierten Lumen deutlich vergrößertes Schlauch- oder sonstiges Leitungslumen mit einem entsprechend reduzierten Fluss-Widerstand angeschlossen werden.

In der Strömungsverbindung lässt sich vorzugsweise extrakorporal ein Filter und/oder eine Dampfbarriere anordnen. Mittels einer Dampfbarriere kann beispielsweise die extrakorporlae Regeleinrichtung vor eindringenger Feuchtigkeit geschützt werden.

Sofern in der Strömungsverbindung extrakorporal ein Konnektor mit einem Innenlumen vorgesehen ist, sollte dessen Innenlumen vorzugsweise über die gesamte Länge des Konnektors im konnektierten Zustand seiner Teilkomponenten eine konstante Querschnittsfläche aufweisen.

Es hat sich bewährt, dass die minimale lichte Innenquerschnittsfläche in der extrakorporalen Strömungsverbindung größer ist als die minimale lichte Innenquerschnittsfläche des in den Tubus oder in einen sonstigen Schaft integrierten oder eingeformten intrakorporalen Strömungskanalabschnitts, beispielsweise wenigstens 1,1-fach so groß wie die minimale lichte Innenquerschnittsfläche des intrakorporalen Strömungskanalabschnitts, vorzugsweise wenigstens 1,2-fach so groß wie die minimale lichte Innenquerschnittsfläche des intrakorporalen Strömungskanalabschnitts, insbesondere wenigstens 1,3-fach so groß wie die minimale lichte Innenquerschnittsfläche des intrakorporalen Strömungskanalabschnitts. Durch eine solche Querschnittserweiterung kann die Strömungsgeschwindigkeit weiter optimiert werden.

Die Erfindung strebt zur durchgängigen Druckstabilisierung im trachealen Cuff auf einen vom Anwender variabel einstellbaren oder durch die spezifische Bauart der Vorrichtung fix vorgegebenen Zielwert im Bereich von 20 bis 40 mbar, bevorzugt von 30 mbar, eine maximale zeitliche Verzögerung von etwa 10 bis 20 Millisekunden an, beginnend vom Zeitpunkt der initialen Auslenkung des thorakalen Drucks bei der aktiven Inspiration des Patienten, aus einer atemmechanischen Ruhelage auf ein Druckniveau unterhalb dieser Ruhelage. Nach maximal 20 Millisekunden (ms) sollen die jeweiligen, von Atemhub zu Atemhub erfolgenden Auslenkungen des Ballondichtungsdrucks auf den Zielwert zurückgeführt werden.

Eine konstruktiv einfache Anordnung erhält man dadurch, dass der Druck in einem Volumenreservoir der extrakorporalen Regeleinrichtung auf den Druck-Sollwert für den ballonartigen Folienkörper eingestellt ist. Solchenfalls ist selbst ohne eine aktive Regelung sichergestellt, dass der Druck in dem Dichtungsballon nicht auf zu große Werte ansteigen kann.

In einem solchen Falle kann in der Strömungsverbindung ein Element mit einer Ventilfunktion und/oder einer flussrichtenden Funktion vorgesehen ist, welches vorzugsweise derart orientiert ist, dass es bei einem Unterdruck in dem ballonartigen Folienkörper gegenüber dem Druck in einem Volumenreseroir der extrakorporalen Regeleinrichtung öffnet und einen schnellen Volumenstrom in den ballonartigen Folienkörper erlaubt, insbesondere auch ohne eine aktive Regelung.

Damit sich ein Überdruck in dem ballonartigen Folienkörper gegenüber dem Druck in einem Volumenreseroir der extrakorporalen Regeleinrichtung allmählich abbauen kann, sollte dem Element mit einer Ventilfunktion und/oder einer flussrichtenden Funktion ein Drosselelement parallelgeschalten sein.

Die für die Druckstabilisierung erforderlichen Volumenverschiebungen zur dichtenden Ballonkomponente werden, im erfindungsgemäßen Verbund eines Dichtungsballons, mit einer in den Schaftschlauch des den Ballon tragenden Katheters integrierten, flussoptimierten Zuleitung zum dichtenden Ballon, durch ein extrakorporales, mit konstantem Druck beaufschlagten Volumenreservoir, oder durch einen, in sonstiger Weise Volumen bei konstantem Druck bereitstellenden, reservoir- und/oder pumpenartigen, permanent wirkenden Mechanismus angetrieben.

Der jeweilige Dichtungsdruck im druckführenden Verbund von Ballon und Zuleitung kann beispielsweise durch einen schwerkraft- oder federkraftbasierten Mechanismus erzeugt und aufrechterhalten werden, der auf eine ballon- oder balgartige Reservoirkomponente, in einer das darin enthaltene Füllmedium, durch Krafteinwirkung auf die äußere Hülle der Reservoirkomponente, komprimierenden bzw. druckbeaufschlagenden Weise einwirkt. Ballon, Zuleitung und Reservoir sind hier zum geschlossenen System verbunden. Die Befüllung und Entleerung des Systems erfolgen über ein, vorzugsweise in das Reservoir integriertes Befüll-Ventil.

Die Beaufschlagung einer Reservoirkomponente mit Druck kann ebenfalls durch einen, permanent mit dem Reservoir konnektierten, pumpenartigen oder elektromechanischen, beispielsweise piezo-elektrisch angetriebenen VentilMechanismus erfolgen.

Alternativ kann der im System herrschende Fülldruck auch durch eine, sich elastisch, in spezifischer Weise, ausdehnender Reservoirblase hergestellt werden. Die Hülle der blasenartigen Komponente geht bei der Befüllung mit einem bestimmten, initialen Volumen in einen Dehnungszustand über, der das in der Blase enthaltene Volumen unter einen baulich, spezifisch determinierten Druck nimmt, der dem gewünschten dichtenden Zieldruck im System entspricht. Bei Steigerung des Füllvolumens der Blase dehnt sich diese in einer charakteristischen "isobaren" Weise weiter auf, wobei der sich in der Reservoirblase einstellende Fehldruck, über einen gewissen Volumenbereich hinweg, nicht steigert bzw. der jeweilige Zieldruck erhalten bleibt. Die Reservoirblase hält somit Volumen in "Vorrat", wobei der Druck des Füllmediums im "isobaren Vorratsbereich" konstant bleibt. Auf eine von extern auf die Reservoirhülle wirkende Kraft kann bei derartigen, beispielsweise aus Polyisopren hergestellten, volumendehnbaren Reservoirblasen verzichtet werden. Eine entsprechende Technik wird beispielsweise in der in WO 2013/139986 A1 beschrieben.

Es sind ferner elektronisch oder elektromechanisch geregelte, mit dem Ballon und der Zuleitung zum Ballon, zu einem kommunizierend geschlossenen System verbundene Komponenten denkbar, die das im Rahmen der Regelung der Ballondichtung benötigte Volumen ohne Zwischenschaltung einer quasi "puffernden", reservoir-artigen Komponente, in Art einer "Quelle", druckkonstant (isobar) in das System fördern bzw. dem System bereitstellen.

Neben solchen druck-konstanten Quellen, die einen Druck generieren, der dem erforderlichen Dichtungsdruck im trachealen Cuff entspricht, können auch regulierende, reservoirartige oder quellenartige Systeme an den Verbund von Ballon und Zuleitung zum Ballon angeschlossen werden, die mit Druckgradienten arbeiten, bzw. die den Druckbereich von 20 bis 40 mbar überschreiten. Solche Systeme halten beispielsweise einen Druck von 100 mbar vorrätig und verschieben das Füllmedium, von relativ zum dichtenden Zieldruck hohen Gradienten angetrieben, in entsprechend beschleunigter Weise. Der zum Cuff gerichtete, beschleunigte Volumenfluss kann beispielsweise durch ein elektronisch gesteuertes Proportionalventil eingestellt werden. Hier bieten insbesondere präzise, piezo-elektrisch angetriebene Ventile eine Grundlage. Die Ventile sind klein, lautlos und haben einen geringen Energieverbrauch. Den Ventilen können zudem piezo-elektrisch angetriebene Pumpen vorgeschaltet werden, die ebenfalls lautlos einen Reservoir Druck von bis zu 100 mbar aufbauen.

Der Druck einer Druckquelle für das Füllmedium in der extrakorporalen Regeleinrichtung, insbesondere stromaufwärts eines regelnden Ventils, lässt sich auf einen Druckwert oberhalb des Sollwertes für den ballonartigen Folienkörper einstellen, beispielsweise auf einen Druckwert von 100 mbar oder mehr, vorzugsweise auf einen Druckwert von 200 mbar oder mehr, bevorzugt auf einen Druckwert von 500 mbar oder mehr, insbesondere auf einen Druckwert von 1 bar oder mehr, oder sogar auf einen Druckwert von 2 bar oder mehr. Der Differenenzdruck zu dem deutlich niedrigeren Druck in dem Dichtungsballon fällt dann zum Großteil über einem regelnden Ventil ab, welches je nach Ausführungsform entweder bei Bedarf nur geringfügig öffnet oder mit einer Taktung betrieben wird, dergestalt, dass der Druck stromabwärts des Ventils geregelt wird.

Zur Erfassung des Druck-Istwertes in dem ballonartigen Folienkörper kann in dem ballonartigen Folienkörper ein Drucksensor angeordnet sein.

Ein solcher Drucksensor in dem ballonartigen Folienkörper sollte über Kabel mit der extrakorporalen Regeleinrichtung verbunden oder verbindbar ist, vorzugsweise wobei das Verbindungskabel innerhalb des Tubus oder des sonstigen Schaftes in einem ggf. zusätzlichen Lumen oder innerhalb der Strömungsverbindung verlegt ist. Daneben wäre natürlich auch eine Funkverbindung denkbar, bspw. über Bluetooth, die aber erheblich aufwendiger ist und eher störanfällig ist als eine Kabelverbindung.

Die Erfindung sieht weiterhin vor, dass die extrakorporale Regeleinrichtung einen aktiven Regler aufweist, vorzugsweise einen elektronischen Regler, insbesondere einen Zweipunktregler, welcher insbesondere derart konzipiert ist, um den als Istwert erfassten Druck innerhalb des ballonförmigen Folienkörpers auf einen vorgegebenen oder vorgebbaren Sollwert möglichst konstant einzuregeln.

Im Verbund mit aktiven, elektronisch gesteuerten Reglern lassen sich besonders optimierte, geschlossene Regelkreise herstellen, wobei druckaufnehmende Sensoren innerhalb der dichtenden Ballonkomponente integriert werden und vorzugsweise über eine Kabelverbindung mit der Steuerung der Reglereinheit verbunden sind. Solche rückgekoppelten Systeme können auch für die aktive Entnahme von Volumen aus einem Ballon ausgelegt sein, wobei vom Regler, parallel zu einem Überdruck-Gradienten, ein Unterdruck-Gradient erzeugt wird, so dass gegebenenfalls Volumen aus dem dichtenden Ballon zum Regler hin gerichtet, in beschleunigter Weise abfließen kann.

Ein im Rahmen der Erfindung verwendeter, elektronischer Zweipunktregler kann mit einer festen Taktfrequenz von beispielsweise zwischen 100 Hz und 1000 Hz betrieben werden, wobei jeweils ein Ventil, beispielsweise ein Piezoventil, zwischen einer Druckquelle für das Füllmedium mit einer entsprechenden Frequenz abwechselnd geöffnet und geschlossen wird, wobei vorzugsweise das Pulsverhältnis zwischen Öffnungs- und Schließphase von dem Regler beeinflusst werden kann, insbesondere als Reaktion auf die Differenz zwischen einem vorgegebenen oder vorgebbaren Druck-Sollwert einerseits und dem innerhalb des ballonartigen Formkörpers gemessenen Druck-Istwert andererseits. Durch das dabei verwendete Regelventil kann es eine durchaus erhebliche Druckdifferenz geben zwischen einem möglichen hohen Druck stromaufwärts eines solchen Ventils, und dem durch die Pulsierung geregelten, niedrigen Druck stromabwärts des Ventils.

Bevorzugt ist das Hohlorgan oder der anatomische Raum die Trachea oder der Ösophagus eines Patienten. Das beschriebene Prinzip der fluss-optimierten, latenzminimierten Volumenkompensation bzw. -stabilisierung in einem tracheal platzierten, dichtenden Ballon kann aber in analoger Weise auch bei der dichtenden Tamponade der Speiseröhre bei einem spontan atmenden Patienten zur Anwendung kommen. Die ebenfalls mit der Eigenatmung des Patienten korrespondierenden Druckschwankungen in einem ösophageal platzierten, in tamponierend dichtender Weise wirkenden Ballon sind in der Regel noch ausgeprägter als bei einem tracheal platzierten Ballon. Sie entsprechen in guter Näherung dem jeweils herrschenden intra-thorakalen Druck. Zur Herstellung einer möglichst effizient dichtenden Ballontamponade in der Speiseröhre schlägt die Erfindung, als bauliche Variante, eine Segmentierung des dichtenden Ballons vor. Während sich das ösophageal dichtende, distale Segment des Ballons über den, sich zwischen dem oberen und unteren Sphinkter erstreckenden Abschnitt der Speiseröhre erstreckt, schließt sich nach proximal ein verjüngtes Ballonsegment an, welches optional bis zum proximalen Ende des den Ballon tragenden Katheters reicht. Der resultierende Spaltraum zwischen Schaft und proximalem Ballonsegment kann großzügig bemessen werden. Er erlaubt die Befüllung des distalen Ballonsegmentes quasi um den Katheterschaft herum und ermöglicht entsprechend fluss-optimierte, rasche Volumenverschiebungen, auch bei kleinen, den Volumenfluss von extrakorporal nach intrakorporal antreibenden Differenzdrucken. Solche dynamisch druckregulierten Ballontamponaden sind insbesondere auf trans-ösophageal platzierten Sonden für die gastrische Ernährung und/oder die Dekompression des Magens anwendbar. Die Anbindung an ein isobar wirkendes Volumenreservoir oder an eine, über eine Differenzdruck regulierte Volumenquelle, kann in einer zur Dichtung der Trachea analogen Ausführung erfolgen.

Die Erfindung sieht weiterhin vor, dass das dichtende Ballonelement aus einer dünnwandigen Ballonfolie aus Polyurethan besteht, welche in dem zur jeweiligen, abzudichtenden Oberfläche hin gerichteten Segment eine Wandungsstärke von 5 bis 30 µm, bevorzugt von 10 bis 20 µm aufweist. Ferner kann das dichtende Ballonelement aus PUR bestehen mit einem Materialdurometer nach Shore von 70 A bis 95A, und/oder mit einem Materialdurometer nach Shore von 54D bis 60D. Ein derartiges Material lässt sich einerseits leicht residual präformieren, so dass es zur Abdichtung nicht in einen elastisch gedehnten Zustand überführt werden muss.

Schließlich entspricht es der Lehre der Erfindung, dass das dichtende Ballonelement einen mehrlagigen Wandaufbau aufweist, wobei mindestens eine Materiallage besondere Barriereeigenschaften für Wasserdampf und/oder Luft aufweist, wobei die Barrierelage beispielsweise aus EVOH besteht. Damit kann bspw. das Eindringen von Feuchtigkeit vermieden werden, die sich in der Zuleitung niederschlagen und dort eine schnelle Volumenverschiebung behindern könnte.

Die folgenden Figuren erläutern die beschriebenen erfinderischen Inhalte anhand konkreter baulicher Ausführungen. Hierbei zeigt:
- Fig. 1: eine beispielhafte Bauform eines erfindungsgemäßen Trachealtubus, der eine Auswahl strömungsoptimierender Komponenten und Merkmale integriert, die auch bei einer kleinen, den Volumenfluss von extrakorporal nach intrakorporal antreibenden Druckdifferenz eine rasche Verschiebung von Füll-Medium zum dichtenden Ballon ermöglichen bzw. eine zur Atemarbeit des Patienten synchrone, sich dynamisch adaptierende Dichtung der Trachea ermöglichen;
- Fig. 2a: einen transversalen Schnitt durch den Schaft eines Trachealtubus, mit einer besonderen Ausführung der in den Schaft integrierten Lumina, wobei die Gesamtquerschnittfläche der in die Wandung des Tubus integrierten Zuleitung zum tracheal dichtenden Cuff optimal groß ausgelegt ist, und wobei durch ein besonderes, hantelförmiges Profil passagere, lumenverschließende Verlegungen der Zuleitung durch in die Zuleitung eingedrungenes Kondenswasser vermieden werden;
- Fig. 2b: eine bauliche Variation der in Fig. 2a dargestellten Ausführungsform, wobei die Zuleitung zum Cuff durch einen separat hergestellten Schlauch erfolgt, der den in Fig 2a beschriebenen hantelförmigen Querschnitt aufweist, jedoch in eine formkongruente Aussparung des dorsalen Tubenschaftes eingelegt und dort fixiert ist;

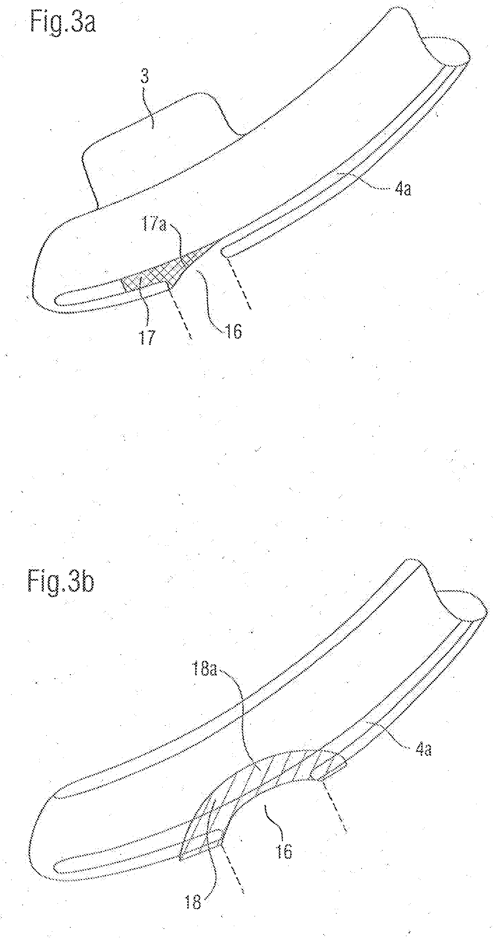

- Fig. 3a: eine Darstellung des distalen, den Ballon tragenden Abschnitts eines erfindungsgemäßen Trachealtubenschaftes, umfassend einen durch eine rampenartige Komponente strömungsoptimierten Übergang von einer in die Wandung des Trachealtubenschaftes integrierten Füll-Leitung in den von der Ballonkomponente umschlossenen Binnenraum;
- Fig. 3b: eine weitere Ausführungsform eines strömungsoptimierten Übergangs von der schaftintegrierten, kanalartigen Zuleitung zum dichtenden Ballon, wobei der Übergang in Form einer schalenartig stützenden Komponente ausgeführt ist, die sich an den äußeren Umfang des Tubenschaftes um den Öffnungsbereich der schaftintegrierten Zuleitung anschmiegt und so die Schaftwandung im Öffnungsbereich stabilisiert bzw. Knickungen des Schaftes vorbeugt;

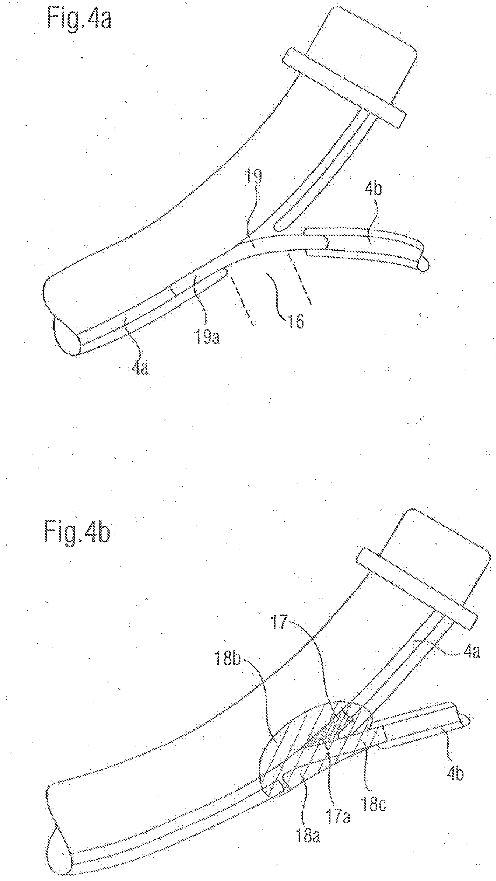

- Fig. 4a: eine Ausführungsform der Erfindung mit einem fluss- bzw. strömungsoptimierten Übergang von einem Befüllschlauch zum schaftintegrierten, kanalartigen Zuleitungslumen;
- Fig. 4b: eine weitere Ausführungsform des Übergangs vom Befüllschlauch zum schaftintegrierten Zuleitungslumen, umfassend eine haubenartige, die Zuleitung und den Befüllschlauch miteinander verbindende Komponente, die zu beiden konnektierten Lumina hin, einen stufenlosen, strömungsoptimierten Übergang herstellt;

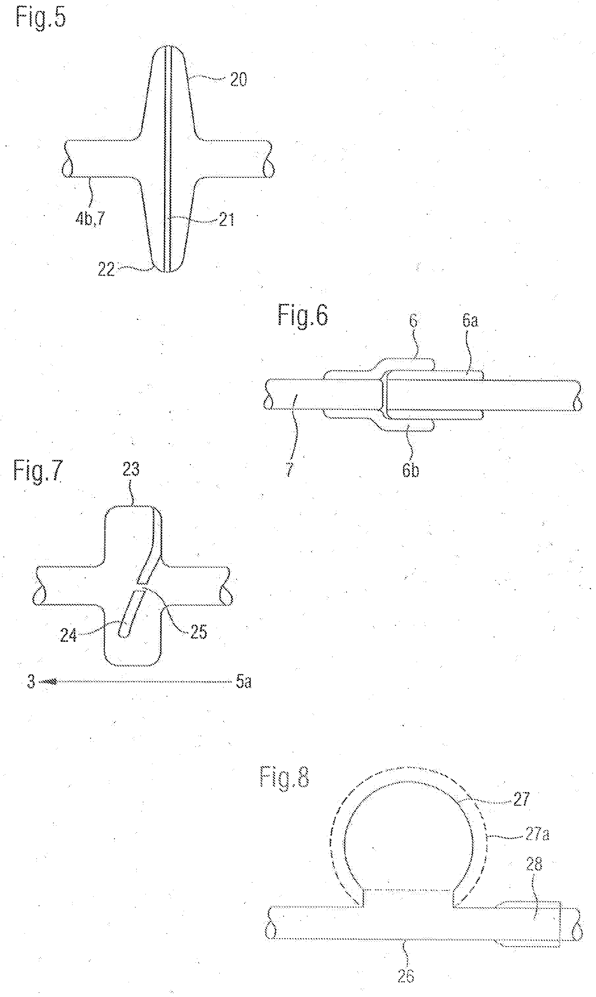

- Fig. 5: eine in den Befüllschlauch integrierte Dampf-Barriere und/oder Mikroben-Barriere in einem Längsschnitt;
- Fig. 6: eine Bauform eines fluss-optimierten Konnektors zwischen dem proximalen Ende des Befüllschlauchs und dem Regler;
- Fig. 7: einen Längsschnitt durch ein leichtgängiges Ventilelement mit optionaler retrograder Volumenausgleichsfunktion;
- Fig. 8: ein extrakorporales Volumenreservoir mit isobarer Volumendehnungscharakteristik;

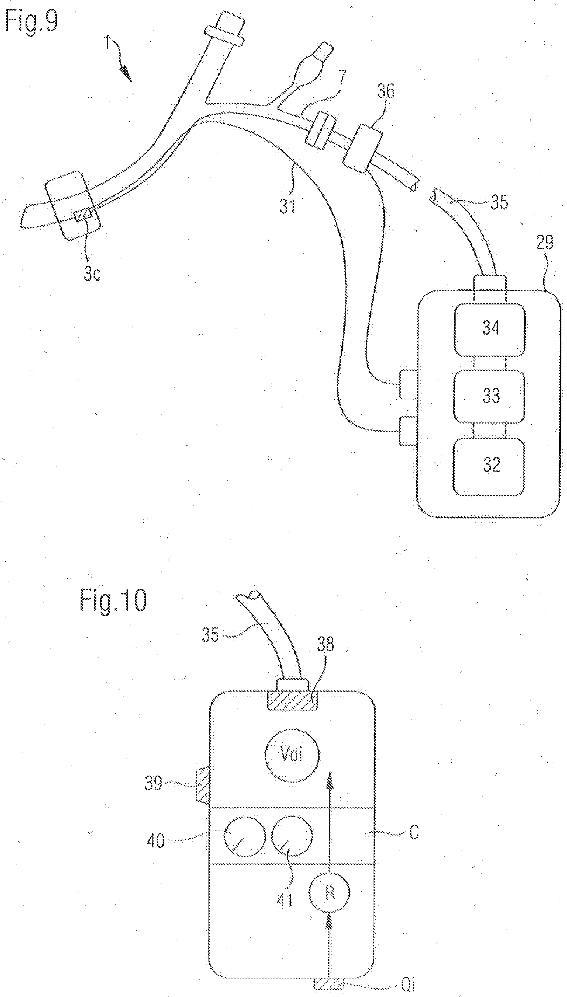

- Fig. 9: ein mit einem tracheal dichtenden Cuff in direkter Weise rückgekoppeltes, elektronisches/elektromechanisches Reglersystem, welches den Zustrom des Füllmediums, und optional auch dessen Abstrom, über die vom Regler zum dichten Cuff hinweg reichende Schlauch- und Kanalzuleitung hinweg, durch einen, flusswiderstände kompensierenden Differenzdruck beschleunigt bzw. wobei der vom Regler erzeugte Druck den Solldruck im tracheal dichtenden Cuff überschreitet, und wobei der Druck im Cuff durch einen innerhalb des Cuffs positionierten, elektronischen Sensor aufgenommen und der Regelereinheit zugeführt wird;
- Fig. 10: ein alternatives, elektronisches/elektromechanisches Reglersystem, wobei die den Cuffdruck aufnehmende elektronische Sensorkomponente in der Reglereinheit integriert ist.

Fig. 1 beschreibt beispielhaft einen erfindungsgemäßen Katheter anhand eines Trachealtubus 1, bestehend aus einem Schaftelement 2, welches am distalen Ende einen tracheal dichtenden Cuff 3 trägt, wobei dieser über eine schaftintegrierte kanalartige Zuleitung 4a, die außerhalb des Schaftes in einen Füllschlauch 4b übergeht, mit einem extrakorporalen Volumenreservoir 5a verbunden ist, welches den Cuff mit Fülldruck beaufschlagt hält bzw. ein gasförmiges Füllmedium mit einem tracheal dichtenden Solldruck vorrätig hält, oder mit einer elektronisch geregelten Volumenquelle 5b verbindet, die den tracheal dichtenden Solldruck durch eine von der Volumenquelle erzeugte Druckdifferenz einstellt bzw. der von der Volumenquelle erzeugte Druck den tracheal dichtenden Solldruck überschreitet. Das Reservoir oder die Quelle wird durch einen Konnektor 6 an den Füllschlauch 4b angeschlossen. Der Füllschlauch 4b weist neben einem Schenkel 7, der mit dem Konnektor 6 abschließt, einen weiteren Schenkel 8 auf, der in einen Pilotballon 9 übergeht, der mit einem Befüll-Ventil 10 versehen ist. Im Schenkel 7 ist optional ein vom Anwender manuell bedienbarer Verschlussmechanismus 11 eingebaut, der die Anbindung der Reservoir- oder Quelleneinheit an einen bereits im Patienten intubierten und mit Fülldruck beaufschlagten Tubus ohne Druckabfall im tracheal dichtenden Cuff ermöglicht. Zur Vermeidung des Übertritts von Kondenswasser aus dem zuleitenden System in die jeweils druckregulierende Einheit ist optional in den Schenkel 7 ein entsprechend gasdurchlässiges Element 12 integriert sein, welches wasserabweisende Eigenschaften aufweist. Alternativ oder ergänzend zur wasserabweisenden Funktion kann das Element 12 auch als Mikrobenfilter wirken. Die Gesamtquerschnittfläche des schaftintegrierten Kanals 4b entspricht einem kreisrunden Querschnitt von mindestens 2 mm, bevorzugt 3 bis 4 mm. Die Querschnittfläche des Innenlumens des Befüll-Schlauchs 4b, sowie des Schenkels 7 und der sich dem Schenkel bis zum Reservoir anschließenden Schlauchleitung entspricht einem kreisrunden Durchmesser, der bevorzugt 2,0 mm überschreitet bevorzugt mehr als 3,0 mm und besonders bevorzugt mehr als 3,5 mm aufweist.

Fig. 2a zeigt eine Ausführung des Schaftelementes 2, wobei die in die Schaftwandung integrierte Zuleitung 4a ein besonderes, im transversalen Schnitt hantelförmiges Profil 13 aufweist, das den Flusswiderstand bestimmenden Gesamtquerschnitt der Zuleitung in optimaler Weise maximiert. Die Zuleitung wird in die dorsale Portion 2a des Tubenschaftes extrudiert, kommt also bei der üblichen Bauweise eines Trachealtubus nach Magill in der sogenannten großen Kurvatur des Schaftes zu liegen, die der breiten Basis des Stimmlippen-Trigonums aufliegt. Das sich durch die hantelförmige Füll-Leitung ergebende Gesamtprofil des Schaftes SQ weist eine entsprechende Verbreiterung der dorsalen "Basis" des Tubenprofils auf. Die breite Basis der großen Kurvatur ermöglicht, dass das ventral im Schaft integrierte, beatmende Lumen 14 des Trachealtubus in seiner runden oder leicht ovalen Form sowie seiner Querschnittfläche erhalten werden kann. Die endständigen Erweiterungen 13a der Befüllleitung liegen jeweils in den äußeren Winkeln der nach dorsal gerichteten Basis des Schaft-Profils. Bei regelrecht intubierter Tubenlage liegt dessen verbreiterte Rückwand der breiten Basis der Glottis in etwa formkongruent an. Die lateralen Erweiterungen des Profils sind durch ein verjüngtes, stegartiges, mittiges Segment 13b verbunden. Die Kommunikation der Erweiterungen durch den mittigen Steg verhindert, dass in die Füll-Leitung eindringendes Kondenswassers einen durch Kapillarkräfte bedingten Verschluss des zum Cuff zuleitenden Lumens verursacht, wodurch die kontinuierliche Verbindung der regelnden Einheit zum Cuff beeinträchtigt werden kann. Ein sich im hantelförmigen Profil ausbildender Flüssigkeitsspiegel reist durch die stegartige Verbindung der einen Profilerweiterung zur parallel angeordneten Erweiterung in der Regel ab, wodurch ein flusswirksamer Verschluss der Füll-Leitung weitgehend ausgeschlossen werden kann. Die Erweiterungen 13a weisen in der bevorzugten Ausführung jeweils einen Durchmesser von 1,5 bis 2,5 mm, bevorzugt von 1,5 bis 2,0 mm auf. Der die Lumina verbindende Steg 13b hat eine Höhe von ca. 0,5 bis 1,0 mm.

Fig. 2b zeigt eine baulich abgewandelte Ausführung des in Fig. 2a beschriebenen Profils 13, wobei die hantelförmige Zuleitung als separat hergestellter Schlauch 15 in eine formkongruente Aussparung 15a in der dorsalen Wandung des Tubus bzw. in dessen große Kurvatur eingelegt und dort fixiert wird. Der Vorteil dieser Bauweise besteht darin, dass der Schlauch oberhalb der Stimmlippenebene aus dem Schaft des Tubus herausgeführt werden kann und ohne strukturelle Unterbrechung als Füllschlauch weitergeführt werden kann. Das Schlauchelement 15 verbindet so den Cuff des Tubus mit den extrakorporal integrierten Elementen der Zuleitung in einer durchgängigen, strömungsoptimierten Weise, wobei flusshemmende Übergänge vermieden werden. Zur Maximierung des Innendurchmessers kann das Schlauchelement 15 einen dünnwandigen, aus einem sich spontan elastisch aufrichtenden Material, wie beispielsweise Polyurethan bestehen, wobei vorzugsweise Materialhärten im Bereich von 90A bis 95A oder 55D bis 60D verwendet werden. Der Tubenschaft selbst besteht vorzugsweise aus sich nichtelastisch, plastisch verformenden PVC.

Fig. 3a stellt den Übergang von der schaftintegrierten Füll-Leitung 4a zum tracheal dichtenden Cuff 3 dar, wobei fluss-hemmende Effekte durch Verwirbelungen beim Übertritt des Füllmediums aus dem zuleitenden Lumen in den Cuff minimiert werden, indem die Umlenkung des Mediums in einem möglichst flachen Winkel erfolgt bzw. ein Übertritt in einem steilen oder rechten Winkel vermieden wird, wie dies bei der herkömmlichen Bauweise von Trachealtuben durch einen einfachen tangentialen Anschnitt der Wandung der Füll-Leitung typischerweise verursacht wird.

Der Anschnitt 16 der Füll-Leitung 4a ist deshalb in seiner axialen Ausdehnung, von 1 bis 2 mm bei konventionellen Tuben, auf 4 bis 10 mm, bevorzugt 5 bis 8 mm verlängert. In die sich nach distal erstreckende Öffnung des Anschnitts der Zuleitung 4a eingeschoben, befindet sich eine die Zuleitung verschließende Komponente 17, die in die Öffnung hinein eine von distal nach proximal absteigende Rampe 17a ausbildet, welche das zum Cuff strömende Medium verwirbelungsfrei in den Cuff lenkt. Die Rampe ersteckt sich über eine axiale Länge von 4 bis 6 mm.

Fig. 3b zeigt eine Komponente 18, die ergänzend zur in Fig. 3a beschriebenen Komponente 17 im Bereich der Öffnung 16 der Zuleitung zur axialen Stabilisierung des Tubenschaftes im Bereich des verlängerten Anschnitts integriert wird. Die Komponente 18 besteht aus besonders dünnwandigem Material hoher Härte, und schmiegt sich der Außenkontur des Anschnitts zirkulär, in sattelartiger Weise auf dessen lateralen Flächen 18a. Die Komponente wird vorzugsweise im Spritzguss hergestellt. Die zuvor beschriebene, lumenverschließende und flusslenkende Komponente 17 kann mit der Komponente 18 baulich verbunden bzw. in diese integriert sein.

Fig. 4a zeigt ein besonderes Übergangselement 19, das die schaftintegrierte Zuleitung 4a in den Füll-Schlauch 4b überleitet bzw. das Lumen der Zuleitung mit dem Lumen des Füll-Schlauchs verbindet. Die Zuleitung 4a ist im Übergangsbereich tangential angeschnitten und über eine Länge von etwa 5mm geöffnet. Das Übergangselement 19 ist bevorzugt aus knickstabilem, dünnwandig ausgeführtem, mit Lösungsmittel verklebbarem Material gefertigt, und weist einen nach distal gerichteten Fortsatz 19a auf, dessen äußere Abmessung in Relation zur Abmessung des Durchmessers der in den Schaft des Tubus integrierten Füll-Leitung vergrößert sind, und so mit einer gewissen Spannung in die durch den tangentialen Anschnitt eröffnete Zuleitung 4a eingeschoben wird. Das Innenlumen des dornartigen Fortsatzes entspricht dem der Zuleitung, wodurch die Formierung stufenartiger Strukturen bzw. ein sich im Übergangsbereich einstellender turbulenter Fluss vermieden werden kann.

Im proximalen Bereich des Übergangselementes 19 weist dieses optional eine hülsenartige Aufnahme für den Füll-Schlauch 4b auf, wobei der Schlauch derart in die Aufnahme eingesteckt und durch Klebung fixiert wird, dass das Innenlumen des Elementes 19 dem Innenlumen des Füll-Schlauches 4b entspricht. Das Element 19 gewährleistet somit auch im Bereich der proximalen Konnektion, dass Kaliberübergänge von der schaftintegrierten Zuleitung zum Füllschlauch vermieden, und flussreduzierende Stufenformationen ausgeschlossen werden. Das Element ermöglicht in diesem besonders fluss-kritischen Bereich einen laminaren Fluss des Füllmediums vom Reservoir bzw. der Quelle zum tracheal dichtenden Cuff. Ferner können durch das Element 19 Übergänge von kreisrunden Querschnitten des Füllschlauchs 4b, zu abgeflachten, ovalen, flach-ovalen, oder auch hantelförmigen Querschnitten des distalen Anteils 19a des Übergangselementes strömungsoptimierend geglättet werden.

Die Querschnittfläche des Füllschlauches 4b entspricht mindestens der Gesamtquerschnittfläche der schaftintegrierten Zuleitung 4a, überschreitet diese aber in den bevorzugten Ausführungsformen.

Fig. 4b zeigt eine haubenartige Komponente 18a, die sich dem dorsalen Umfang der Trachealtubus passgenau anschmiegt und den Anschnitt der Zuleitung 4a dicht schließend abdeckt. Die Komponente weist laterale, sattelartig flächige Ausläufer 18b auf, die sich mit der lateralen Wandung des Tubenschaftes, in einer den Tubenschaft im Bereich des Anschnitts stabilisierenden Weise verbinden. Die haubenartige Komponente weist einen, im flachen Winkel nach proximal abgehenden Stutzen 18c auf, der den Füllschlauch 4b aufnimmt. Die Figur zeigt ferner eine Komponente 17, die in den Zuleitungskanal 4a nach proximal gerichtet eingeschoben wird, und diesen verschließt. Analog zur rampenartigen Bauweise in Fig. 3a, formiert der Stutzen 18 eine fluss-optimierende Rampe 17a, die von proximal nach distal in den Anschnitt der Zuleitung reicht, und sich dabei entsprechend abschrägt.

Fig. 5 zeigt eine optionale, in den Füllschlauch 4b oder den Schenkel 7 integrierte Dampfbarriere 20. Die Fläche der jeweiligen Trennlage 21 ist dabei derart groß gewählt, dass der durch die Barrierefunktion verursachte Flusswiderstand kompensiert und Verzögerungen beim Druckausgleich im Cuff vermieden wird. Die Barriere-Lage weist einen Durchmesser von mindestens 10 bis maximal 25 mm, bevorzugt 15 mm auf. Das Gehäuse 22 ist flach und diskoid entworfen und reduziert so den residualen Raum um die Trennlage. Die Funktion der Dampfbarriere stellt sicher, dass Wasserdampf und Kondensat nicht in den Bereich der regelenden Einheit vordringen und dort zu einer Beeinträchtigung des Schließ- und Öffnungsverhaltens dort verbauter Ventile führen. Alternativ oder auch in Ergänzung zur Dampfbarriere 20 kann auch eine mikrobendichte Barriere-Lage in das Gehäuse 22 verbaut sein.

Fig. 6 zeigt einen besonderen Konnektor 6, der über sein gesamtes Innenlumen eine konstante Querschnittfläche aufweist. Durch die Steckung des männlichen Teils 6a in den weiblichen Teil 6b wird so ein stufenloser, spaltloser und gratfreier Übergang ermöglicht. Die Querschnitte der miteinander konnektierten Lumina sind im Bereich der Konnektion identisch.

Fig. 7 zeigt eine Einheit 23 mit Ventilfunktion, wobei die flussrichtende Funktion vorzugsweise durch ein lappenartiges, folienartig dünnes Ventilplättchen 24 hergestellt wird. Im geöffneten Zustand des Ventils stellt sich eine QuerschnittFläche der Ventilöffnung ein, die mindestens dem Durchmesser der distal zum Ventil angeordneten Leitung entspricht, bevorzugt diese aber überschreitet. Das Ventilplättchen verfügt bevorzugt über eine lochartige Perforation 25, die auch im geschlossenen Zustand des Ventils einen reduzierten, dem Hauptstrom entgegengerichteten Volumenstrom vom Cuff zur regelnden Einheit ermöglicht, so dass passagere Überdrucksituationen im Cuff durch einen entsprechend verzögerten Volumenabfluss aus dem Cuff ausgeglichen werden können.

Eine entsprechend wirkende Rückstrom-Funktion kann alternativ durch ein parallel zum Ventil-Plättchen angeordnete, kanalartig Verbindung erfolgen, welche einen gewissen, gedrosselten Abstrom von Medium vom Cuff zum Reservoir bzw. zur Quelle ermöglicht.

Fig. 8 zeigt ein Volumenreservoir 26, das über eine spezifisch volumendehnbare Reservoirblase 27 verfügt, die über einen bestimmten radialen Expansionsbereich 27a der Blasenhülle hinweg, das Füllmedium bei einem konstanten, isobaren Druck aufnimmt. Der sich bei der Dehnung der Blasenhülle einstellende Plateaudruck wird durch die spezifische Bauweise bzw. das verwendete Material und die Geometrie der Blase definiert. Der jeweilige Plateaudruck im Reservoir entspricht dem im Ballon herrschenden Solldruck, von beispielsweise 30 mbar. Der von der expandierten Reservoirblase erzeugte Druck treibt im Moment eines Druckabfalls Ballon den Volumenstrom zum dichtenden Ballon-Element an. Das Reservoir kann durch ein Füllventil 28 mit vorzugsweise mit Luft als Füllmedium befüllt werden.

Fig. 9 zeigt ein rückgekoppeltes Reglersystem 29, das an einen fluss-optimierten Katheter 1 des erfindungsgemäßen Bautyps angeschlossen ist. Der Katheter verfügt innerhalb des dichtenden Ballons über einen dort fix integrierten elektronischen Drucksensor 30, der über eine Kabelverbindung 31 mit dem Reglersystem verbunden wird. Der Regler selbst besteht aus einem Pumpen-Modul 32 mit optional integrierten Reservoir 33 und mindestens einem regelnden Ventil-Modul 34 mit integrierter Steuereinheit. Soll- und Alarmwerte können vom Anwender in die Steuerung eingegeben werden. Optional kann der Regler auch über zwei Pumpensysteme, mit jeweils angeschlossenem Reservoir verfügen, wobei das eine Reservoir einen Überdruck und das andere einen Unterdruck vorrätig hält. Die Gradienten bzw. der im Regler vorrätig gehaltene Differenzdruck zum Sollwert im Cuff werden vom Regler in einer optionalen Ausführung selbständig, durch einen lernenden Algorithmus derart eingestellt, dass die Latenz bis zum Erreichen des Sollwertes im Cuff im Bereich von 10 bis 20 ms liegt. Sowohl die Pumpfunktionen als auch die Ventilfunktionen basieren bevorzugt auf piezo-elektrischen Komponenten, die präzise und schnell, sowie leise und energiesparend betrieben werden können.

Die Zuleitung 35 zum Schenkel 7 des Katheters weist bevorzugt einen inneren Durchmesser auf, der den Durchmesser des Schenkels überschreitet, und im idealen Fall um 30% überschreitet, um so widerstandsbedingte Strömungsverluste möglichst klein zu halten. Alternativ zum cuff-integrierten Druck-Sensor, kann ein peripherer druckwandelnder Sensor 36 in die Zuleitung integriert sein, der in unmittelbarer Nähe zum Konnektor 6 positioniert ist. Bei dieser Ausführung kann auf einen im Cuff integrierten Sensor verzichtet werden, wobei eine gewisse Verzögerung der Regelzeit in Kauf genommen wird.

Fig. 10 beschreibt eine weitere Reglereinheit 37 zur kontinuierlichen Aufrechterhaltung eines dichtenden Ballonfülldrucks. Der Cuff des Trachealtubus wird bereits bei der Herstellung auf sein erforderliches Arbeitsmaß ausgeformt. Die Befüllung des Ballons erfolgt in der zuvor beschriebenen, flussoptimierten bzw. widerstandsminimierten Weise. Der Ballon wird mit gasförmigen Medium befüllt. Die Schlauch-Zuleitung 35 vom Regler zum Konnektor 6 sollte ein kreisrundes Lumen von mindestens 5 mm Durchmesser aufweisen, um flussbedingte Druckverluste und dämpfende Effekte zwischen Ballon und Regler zu vermeiden. Die Reglereinheit besteht aus einem einzigen piezo-elektrisch arbeitenden Ventil Voi, das sowohl Volumen zum tamponierenden Ballon richtet als auch Volumen aus diesem ablässt. Dem Ventil patientenseitig vorgeschaltet ist eine elektronisch druckmessende Komponente 38, die den Druck im Ballonkorpus kontinuierlich erfasst und der Steuereinheit C zuführt. Die Reglereinheit verfügt über keine Messfunktion eines unmittelbar im Ballon elektronisch gemessenen Drucks. Dem Ventil ist auf der patientenabgewandten Seite ein im Gerät integriertes Druckreservoir R oder eine externe Druckquelle Qi vorgeschaltet, die Füllmedium beispielsweise im Druckbereich von 1 bis 2 bar vorrätig hält. Das Piezo-Ventil Voi reduziert diesen Vorratsdruck auf ca. 10 bis 30 mbar Dichtungsdruck im Ballon. Kommt es zu einer Überschreitung des vom Anwender eingestellten Solldrucks im Ballon, wird dieser von der Komponente 38 erfasst. Die Steuereinheit C öffnet dann das Ventil Voi, lässt dann Füllmedium, dem jeweiligen Gradienten folgend, über eine Öffnung 39 zur Umgebung hin ab. Die Einheit verfügt über eine Einstellmöglichkeit des tracheal dichtenden Solldruckes 40 und eine Möglichkeit der Einstellung des jeweiligen Volumenflusses 41 zum oder vom Ballon.

**Bezugszeichenliste**

| | | | |
|---|---|---|---|
| 1 | Trachealtubus | 17 | Komponente |
| 2 | Schaftelement | 17a | Rampe |
| 2a | rückwandige Portion | 18 | Komponente |
| 3 | Cuff | 18a | laterale Fläche |
| 4a | kanalartige Zuleitung | 18b | flächiger Ausläufer |
| 4b | Füllschlauch | 19 | Übergangselement |
| 5a | Volumenreservoir | 19a | distaler Anteil |
| 5b | Volumenquelle | 20 | Dampfbarriere |
| 6 | Konnektor | 21 | Trennlage |
| 6a | männliches Teil | 22 | Gehäuse |
| 6b | weibliches Teil | 23 | Einheit |
| 7 | Schenkel | 24 | Ventilplättchen |
| 8 | Schenkel | 25 | lochartige Perforation |
| 9 | Pilotballon | 26 | Volumenreservoir |
| 10 | Befüllventil | 27 | Reservoirblase |
| 11 | Verschlussmechanismus | 27a | Füllbereich |
| 12 | gasdurchlässiges Element | 28 | Füllventil |
| 13 | hantelförmiges Profil | 29 | Reglersystem |
| 13a | endständige Erweiterung | 30 | Drucksensor |
| 13b | mittiges Segment | 31 | Kabelverbindung |
| 14 | beatmendes Lumen | 32 | Pumpen-Modul |
| 15 | Schlauch | 33 | Reservoir |
| 15a | formkongruente Aussparung | 34 | Ventil-Modul |
| 16 | Anschnitt | 35 | Zuleitung |
| 16a | distale Öffnung | 36 | Sensor |
| 37 | Reglereinheit | | |
| 38 | Komponente | | |
| 39 | Öffnung | | |
| 40 | Solldruck | | |
| 41 | Volumenfluss | | |
| C | Steuereinheit | | |
| Qi | externe Druckquelle | | |
| R | Druckreservoir | | |
| SQ | Schaftprofil | | |
| Voi | Ventil | | |

## Patentansprüche

1. Vorrichtung für die organsynchron volumenkompensierende Dichtung eines Hohlorgans oder eines anatomische Raumes mit einem vorgegebenen anatomischen Maß, umfassend (i) einen intrakorporalen, ballonartigen Folienkörper (3) mit dichtenden Flächen zur Anlage an der Wandung des jeweiligen Hohlorgans oder Raumes, während der ballonartige Folienkörper (3) selbst mit einem Füllmedium unter einem Solldruck von höchstens 50 mbar befüllt ist, vorzugsweise unter einem Solldruck von höchstens 40 mbar, insbesondere unter einem Solldruck von höchstens 30 mbar, (ii) einen Tubus (1) oder sonstigen Schaft, welchem der ballonartige Folienkörper (3) aufsitzt, (iii) eine extrakorporale Regeleinrichtung (29) mit einem Volumenreservoir (26,R) und/oder einer Druckquelle (Qi) für das Füllmedium, sowie (iv) eine Strömungsverbindung (4a,4b,7,13,15,35) zwischen dem intrakorporalen ballonartigen Folienkörper (3) und der extrakorporalen Regeleinrichtung (29), die zumindest bereichsweise in oder entlang des Tubus (1) oder sonstigem Schaft verläuft, wobei in der Strömungsverbindung (4a,4b,7,13,15,35) extrakorporal ein Konnektor (6) vorgesehen ist, **dadurch gekennzeichnet, dass**
a) der ballonartige Folienkörper (3) auf ein residuales, das anatomische Maß des Organs oder des jeweiligen Raumes überschreitendes Maß ausgeformt ist, derart, dass die dichtenden Flächen des ballonartigen Folienkörpers (3) der Wandung des jeweiligen Hohlorgans oder Raumes bei spannungslos ungedehntem ballonartigen Folienkörper (3) zumindest bereichsweise unter Ausbildung von Einfaltungen anliegen, und dass
b) die Strömungsverbindung (4a,4b,7,13,15,35)
b1) zwischen dem intrakorporalen ballonartigen Folienkörper (3) und der extrakorporalen Regeleinrichtung (29) in dem Bereich ihres Verlaufs in oder entlang des Tubus (1) oder sonstigen Schaftes einschließlich eines Übergangsbereichs von dem Tubus (1) oder sonstigen Schaft zu einem davon losgelösten Verlauf frei von rechtwinkligen Umlenkungen ist, so dass sich dort eine laminare Strömung ausbilden kann und innerhalb einer Latenzzeit von 200 ms oder weniger, beispielsweise von 100 ms oder weniger, vorzugsweise von 50 ms oder weniger, insbesondere von 25 ms oder weniger, die in dem ballonartigen Folienkörper (3) benötigte zusätzliche Füllmenge des Füllmediums ergänzt werden kann, um Schwankungen des Ballonfülldrucks und/oder des Ballonvolumens und/oder der am ballonartigen Folienkörper (3) anlastenden Drucke und Kräfte zu kompensieren, so dass die Dichtung oder die raumfüllende Tamponade des Hohlorgans oder Raums unter dynamisch alternierenden Schwankungen des Ballonfülldrucks bei einem Druckabfall in dem ballonartigen Folienkörper (3) um 30 mbar erhalten bleibt, und
b2) im Bereich des Übergangs zwischen verschiedenen Komponenten (4a,4b,7,13,15,35), insbesondere im Bereich des Konnektors (6), frei von Knicken ist und frei von Kanten und frei von Abstufungen und frei von Spalten und frei von Graten und frei von sonstigen abrupten Erhebungen oder Vertiefungen, um die laminare Strömung nicht zu beeinträchtigen.

2. Vorrichtung nach Anspruch 1, **dadurch gekennzeichnet, dass** die Strömungsverbindung (4a,4b,7,13,15,35) im Bereich des Tubus (1) oder des sonstigen Schaftes
a) frei von Knicken ist und/oder frei von Kanten und/oder frei von Abstufungen und/oder frei von Spalten und/oder frei von Graten und/oder frei von sonsten abrupten Erhebungen oder Vertiefungen, um die laminare Strömung nicht zu beeinträchtigen, und/oder
b) frei von Biegungen ist, deren Biegeradius in Längsrichtung der Strömung kleiner ist als 0,5 cm, beispielsweise kleiner als 1 cm, vorzugsweise kleiner als 2 cm, insbesondere kleiner als 5 cm.

3. Vorrichtung nach einem der vorhergehenden Ansprüche, **dadurch gekennzeichnet, dass** sich die Querschnittsfläche der Strömungsverbindung (4a,4b,7,13,15,35)
a) von dem Bereich des Tubus (1) oder des sonstigen Schaftes ausgehend bis hin zu der extrakorporalen Regeleinrichtung (29) nicht verringert, und/oder
b) in dem Übergangsbereich von dem Tubus (1) oder sonstigen Schaft zu einem davon losgelösten Verlauf vergrößert, und/oder
c) dass die minimale lichte Innenquerschnittsfläche in der extrakorporalen Strömungsverbindung (4b,7,13,15,35) größer ist als die minimale lichte Innenquerschnittsfläche des in den Tubus (1) oder in einen sonstigen Schaft integrierten oder eingeformten intrakorporalen Strömungskanalabschnitts (4a), beispielsweise wenigstens 1,1-fach so groß wie die minimale lichte Innenquerschnittsfläche des intrakorporalen Strömungskanalabschnitts (4a), vorzugsweise wenigstens 1,2-fach so groß wie die minimale lichte Innenquerschnittsfläche des intrakorporalen Strömungskanalabschnitts (4a), insbesondere wenigstens 1,3-fach so groß wie die minimale lichte Innenquerschnittsfläche des intrakorporalen Strömungskanalabschnitts (4a).

4. Vorrichtung nach einem der vorhergehenden Ansprüche, **dadurch gekennzeichnet, dass** der Querschnitt der Strömungsverbindung (4a,4b,7,13,15,35) in oder entlang des Tubus (1) oder sonstigen Schaftes einen bogenförmige Gestalt aufweist, die sich vorzugsweise einem funktionellen Lumen innerhalb des Tubus (1) oder sonstigen Schaftes etwa tangential anschmiegt oder jenes koaxial umgibt.

5. Vorrichtung nach einem der vorhergehenden Ansprüche, **dadurch gekennzeichnet, dass** die Strömungsverbindung (4a,4b,7,13,15,35) im Bereich des Tubus (1) oder sonstigen Schaftes als jenem an dessen Außenseite anfügbare Leitung oder Schlauchleitung ausgebildet ist, vorzugsweise wobei in der Außenseite des Tubus (1) oder sonstigen Schaftes eine muldenförmige Rinne oder Vertiefung eingeformt ist zur Aufnahme einer anfügbaren Leitung oder Schlauchleitung, insbesondere wobei die muldenförmige Rinne oder Vertiefung seitliche Hinterschneidungen aufweist, so dass eine dort eindrückbare oder einsteckbare Leitung oder Schlauchleitung festgehalten wird und sich nicht selbsttätig lösen kann, und/oder wobei gegebenenfalls die dem Tubus (1) oder sonstigen Schaft an dessen Außenseite anfügbare Leitung oder Schlauchleitung derart präformiert ist, dass sie die muldenförmige Rinne oder Vertiefung ausfüllt und dabei die angrenzenden Außenkonturen des Tubus (1) oder sonstigen Schaftes konturerhaltend ergänzt.

6. Vorrichtung nach einem der vorhergehenden Ansprüche, **dadurch gekennzeichnet, dass** in dem Übergangsbereich der Strömungsverbindung (4a,4b,7,13,15,35) von einem in den Tubus (1) oder in einen sonstigen Schaft integrierten oder eingeformten Strömungskanalabschnitt (4a) zu einem davon losgelösten Verlauf des Strömungskanals (4b,7,13,15,35)
a) eine Komponente (17,17a) mit einem rampen- oder bogenförmigen Verlauf vorgesehen, insbesondere eingefügt ist, vorzugsweise wobei eine lösbare Komponente (17,17a) mittels eines rückwärtigen, an einer der Rampe (17a) abgewandten Seite angeordneten, vorzugsweise dornartigen Fortsatz (17) in eine mit dem in den Tubus (1) oder in einen sonstigen Schaft integrierten oder eingeformten Strömungskanalabschnitt (4a) fluchtende Vertiefung eingesteckt ist, und/oder
b) eine Komponente (18) aus einem dünnwandigen, sich der Mündung des Strömungskanals (4a) in dem Tubus (1) oder sonstigen Schaft anschmiegenden Material eingefügt ist, und/oder
c) eine Komponente (19) mit einer rohrförmigen Gestalt und einem sanft gebogenen Verlauf aus einem knickstabilen Material eingefügt ist, vorzugsweise wobei der Außenquerschnitt der Komponente (19) von rohrförmiger Gestalt größer ist als der Innenquerschnitt des in den Tubus (1) oder in einen sonstigen Schaft integrierten oder eingeformten Strömungskanalabschnitts (4a), vorzugsweise derart, dass er dort unter lokaler Aufdehnung des Strömungskanalabschnitts (4a) reibschlüssig festlegbar ist, und/oder
d) eine haubenförmige Komponente (18a) mit lateralen, sattelartig flächigen Ausläufern (18b) an- oder eingefügt ist, wobei sich die Ausläufer (18b) vorzugsweise mit dem davon abgedeckten Tubenschaft stabilisierend verbinden lassen, beispielsweise adhäsiv, wobei gegebenenfalls eine Komponente (17,17a) mit einem rampen- oder bogenförmigen Verlauf von einer haubenförmigen Komponente (18a) abgedeckt ist, vorzugsweise wobei eine in dem Übergangsbereich der Strömungsverbindung (4a,4b,7,13,15,35) von einem in den Tubus (1) oder in einen sonstigen Schaft integrierten oder eingeformten Strömungskanalabschnitt (4a) zu einem davon losgelösten Verlauf des Strömungskanals (4b,7,13,15,35) angeordnete Komponente (17,17a,18,18a,19) im Bereich ihres proximalen Endes mit einer Muffe zum Auf- oder Einstecken eines Schlauchs versehen ist.

7. Vorrichtung nach einem der vorhergehenden Ansprüche, **dadurch gekennzeichnet, dass**
a) in der Strömungsverbindung (4a,4b,7,13,15,35) vorzugsweise extrakorporal ein Filter und/oder eine Dampfbarriere (20) vorgesehen ist, und/oder
b) dass das Innenlumen in dem Konnektor (6) über die gesamte Länge des Konnektors (6) im konnektierten Zustand von dessen Teilkomponenten eine konstante Querschnittsfläche aufweist.

8. Vorrichtung nach einem der vorhergehenden Ansprüche, **dadurch gekennzeichnet, dass** der Druck in einem Volumenreseroir (26,R) der extrakorporalen Regeleinrichtung (29) auf den Druck-Sollwert für den ballonartigen Folienkörper (3) eingestellt ist.

9. Vorrichtung nach einem der vorhergehenden Ansprüche, **dadurch gekennzeichnet, dass** in der Strömungsverbindung (4a,4b,7,13,15,35) ein Element (23) mit einer Ventilfunktion und/oder einer flussrichtenden Funktion vorgesehen ist, welches vorzugsweise derart orientiert ist, dass es bei einem Unterdruck in dem ballonartigen Folienkörper (3) gegenüber dem Druck in einem Volumenreseroir (26,R) der extrakorporalen Regeleinrichtung (29) öffnet und einen schnellen Volumenstrom in den ballonartigen Folienkörper (3) erlaubt, insbesondere auch ohne eine aktive Regelung, vorzugsweise wobei dem Element (23) mit einer Ventilfunktion und/oder einer flussrichtenden Funktion ein Drosselelement parallelgeschalten ist, insbesondere derart, dass sich ein Überdruck in dem ballonartigen Folienkörper (3) gegenüber dem Druck in einem Volumenreseroir (26,R) der extrakorporalen Regeleinrichtung (29) allmählich abbauen kann.

10. Vorrichtung nach einem der vorhergehenden Ansprüche, **dadurch gekennzeichnet, dass** der Druck einer Druckquelle (Qi) für das Füllmedium in der extrakorporalen Regeleinrichtung (29), insbesondere stromaufwärts eines regelnden Ventils, auf einen Druckwert oberhalb des Sollwertes für den ballonartigen Folienkörper (3) eingestellt ist, beispielsweise auf einen Druckwert von 100 mbar oder mehr, vorzugsweise auf einen Druckwert von 200 mbar oder mehr, bevorzugt auf einen Druckwert von 500 mbar oder mehr, insbesondere auf einen Druckwert von 1 bar oder mehr, oder sogar auf einen Druckwert von 2 bar oder mehr.

11. Vorrichtung nach einem der vorhergehenden Ansprüche, **dadurch gekennzeichnet, dass** in dem ballonartigen Folienkörper (3) ein Drucksensor angeordnet ist, so dass der Druck-Istwert in dem ballonartigen Folienkörper (3) erfasst werden kann, vorzugsweise wobei der Drucksensor in dem ballonartigen Folienkörper (3) über Kabel mit der extrakorporalen Regeleinrichtung (29) verbunden oder verbindbar ist, vorzugsweise wobei das Verbindungskabel innerhalb des Tubus (1) oder sonstigen Schaftes in einem ggf. zusätzlichen Lumen oder innerhalb der Strömungsverbindung (4a) verlegt ist.

12. Vorrichtung nach einem der vorhergehenden Ansprüche, **dadurch gekennzeichnet, dass** die extrakorporale Regeleinrichtung (29) einen aktiven Regler aufweist, vorzugsweise einen elektronischen Regler, insbesondere einen Zweipunktregler, welcher insbesondere derart konzipiert ist, um den als Istwert erfassten Druck innerhalb des ballonförmigen Folienkörpers (3) auf einen vorgegebenen oder vorgebbaren Sollwert möglichst konstant einzuregeln, vorzugsweise wobei der Zweipunktregler mit einer festen Taktfrequenz von beispielsweise zwischen 100 Hz und 1000 Hz betrieben wird, wobei jeweils ein Ventil, beispielsweise ein Piezoventil, zwischen einer Druckquelle (Qi) für das Füllmedium mit einer entsprechenden Frequenz abwechselnd geöffnet und geschlossen wird, wobei vorzugsweise das Pulsverhältnis zwischen Öffnungs- und Schließphase von dem Regler beeinflusst werden kann, insbesondere als Reaktion auf die Differenz zwischen einem vorgegebenen oder vorgebbaren Druck-Sollwert einerseits und dem innerhalb des ballonartigen Folienkörpers (3) gemessenen Druck-Istwert andererseits.

13. Vorrichtung nach einem der vorhergehenden Ansprüche, **dadurch gekennzeichnet, dass** die dichtenden Flächen des ballonartigen Folienkörpers (3) der Wandung des jeweiligen Organs oder Raumes mit einem möglichst konstant wirkenden Dichtungsdruck des ballonartigen Folienkörpers (3) allseitig dichtend und/oder in einer einen ggf. verbleibenden Restraum zwischen dem ballonartigen Folienkörper (3) und einer angrenzender Struktur möglichst minimierenden Weise anliegen.

14. Vorrichtung nach einem der vorhergehenden Ansprüche, **dadurch gekennzeichnet, dass** bei einem Trachealtubus (1)
a) eine definiert großlumige, flussoptimierende Zuführung des Füllmediums zum Trachealtuben-Cuff (3) vorgesehen ist, und/oder
b) die Länge einer schaft-integrierten Zuleitung (4a) zum dichtenden ballonartigen Folienkörper (3) auf ein Minimum reduziert ist, vorzugsweise derart, dass der bauliche Übergangsbereich von dem Tubus (1) oder Schaft zu dem Befüllschlauch (4b) etwa 1 bis 2 cm oberhalb der Stimmlippenebene (Glottis) liegt, und/oder
c) ein turbulenter Fluss beim Verschieben des Füllmediums zwischen Reservoir oder Regler (35) und dem Cuff (3) vermieden ist, und/oder
d) im Bereich des Übergangs vom schaft-integrierten Lumen (4b) in den tracheal dichtenden Cuff (3), und/oder im Bereich des Übergangs vom schaft-integrierten Zuleitungs-Lumen (4a) in den nach extrakorporal reichenden Zuleitungsschlauch (4b), sowie zwischen den Anteilen des Konnektors (6) die Flusseigenschaften dahingehend optimiert sind, dass im dichtenden Ballonelement (3) eine dichtungsdruckerhaltende, extrakorporale Volumenkompensation zeitsynchron wirkend erreicht werden kann, und/oder
e) zwischen den Anteilen des Konnektors (6), und/oder im Bereich integrierter Filter- oder Ventil-Komponenten (20,23) die Flusseigenschaften dahingehend optimiert sind, dass im dichtenden Ballonelement (3) eine dichtungsdruckerhaltende, extrakorporale Volumenkompensation zeitsynchron wirkend erreicht werden kann.

15. Vorrichtung nach einem der vorhergehenden Ansprüche, **dadurch gekennzeichnet, dass** das dichtende Ballonelement bzw. der ballonartige Folienkörper (3)
a) aus einer dünnwandigen Ballonfolie aus Polyurethan besteht, welche in dem zur jeweiligen, abzudichtenden Oberfläche hin gerichteten Segment eine Wandungsstärke von 5 bis 30 µm, bevorzugt von 10 bis 20 µm aufweist, und/oder
b) aus PUR besteht mit einem Materialdurometer nach Shore von 70 A bis 95A, und/oder mit einem Materialdurometer nach Shore von 54D bis 60D, und/oder
c) einen mehrlagigen Wandaufbau aufweist, wobei mindestens eine Materiallage besondere Barriereeigenschaften für Wasserdampf und/oder Luft aufweist, wobei die Barrierelage beispielsweise aus EVOH besteht.

## Claims

1. A device for the volume-compensating sealing of a hollow organ or an anatomical space in sync with organs, comprising (i) an intracorporeal balloon-type foil body (3) formed to a specified dimension, having sealing surfaces, which contact the wall of the respective hollow organ or space, while the balloon-type foil body is itself filled with a filling medium under a maximum target pressure of 50 mbar, preferably under a maximum target pressure of 40 mbar, in particular under a maximum target pressure of 30 mbar, (ii) a tube (1) or other shaft, on which rests the balloon-type foil body (3), (iii) an extracorporeal regulating device (29) with a volume reservoir (26, R) and/or a pressure source (Qi) for the filling medium, as well as (iv) a flow connection (4a, 4b, 7, 13, 15, 35) between the intracorporeal balloon-type foil body (3) and the extracorporeal regulating device (29), which extends at least in regions in or along the tube (1) or other shaft, whereby a connector (6) is provided extracorporeally in the flow connection (4a, 4b, 7, 13, 15, 35), **characterized in that**
a) a balloon-type foil body (3) is formed to a residual dimension, exceeding the anatomical dimension of the organ or the respective space so that the sealing surfaces of the balloon-type foil body (3) contact the wall of the respective hollow organ or space at least in regions while forming folds, when the unexpanded balloon-type foil body (3) is free of tension; and that
b) the flow connection (4a, 4b, 7, 13, 15, 35)
b1) between the intracorporeal balloon-type foil body (3) and the extracorporeal regulating device (29) is free of right-angled deflections in the region of its extension in or along the tube (1) or other shaft including a transition region from the tube (1) or other shaft to an extension that is detached therefrom, so that a laminar flow can form there, and the additional filling quantity of the filling medium needed in the balloon-type foil body (3) can be supplemented within a latency period of 200 ms or less, for example of 100 ms or less, preferably of 50 ms or less, in particular of 25 ms or less, in order to compensate for fluctuations of the balloon filling pressure and/or of the balloon volume and/or of the pressures and forces bearing on the balloon-type foil body (3), so that the sealing or the space-filling tamponade of the hollow organ or of the space is maintained under dynamically alternating fluctuations of the balloon filling pressure with a pressure drop in the balloon-type foil body (3) of 30 mbar, and
b2) is free of kinks and/or free of edges and/or free of steps and/or free of gaps and/or free of ridges and/or free of other abrupt elevations or depressions in the region of a transition between different components (4a, 4b, 7, 13, 15, 35), especially in the region of connector (6), so as not to impair the laminar flow.

2. The device according to claim 1, **characterized in that** the flow connection (4a, 4b, 7, 13, 15, 35) in the region of the tube (1) or of the other shaft
a) is free of kinks and/or free of edges and/or free of steps and/or free of gaps and/or free of ridges and/or free of other abrupt elevations or depressions, so as not to impair the laminar flow, and/or
b) is free of bends, whose bending radius in the longitudinal direction of the flow is less than 0,5 cm, for example less than 1 cm, preferably less than 2 cm, in particular less than 5 cm.

3. The device according to one of the preceding claims, **characterized in that** the cross-sectional area of the flow connection (4a, 4b, 7, 13, 15, 35)
a) does not decrease starting from the region of the tube (1) or of the other shaft up till the extracorporeal regulating device (29); and/or
b) increases in the transition region from the tube (1) or other shaft to a progression that is detached therefrom; and/or
c) **in that** the minimum clear inside cross-sectional area in the extracorporeal flow connection (4b, 7, 13, 15, 35) is larger than the minimum clear inside cross-sectional area of the intracorporeal flow channel section (4a) formed in or integrated into the tube (1) or in another shaft, for example at least 1.1 times as large as the minimum clear inside cross-sectional area of the intracorporeal flow channel section (4a), preferably at least 1.2 times as large as the minimum clear inside cross-sectional area of the intracorporeal flow channel section (4a), in particular at least 1.3 times as large as the minimum clear inside cross-sectional area of the intracorporeal flow channel section (4a).

4. The device according to one of the preceding claims, **characterized in that** the cross section of the flow connection (4a, 4b, 7, 13, 15, 35) in or along the tube (1) or other shaft comprises an arch-shaped form, which preferably tangentially nestles a functional lumen inside the tube (1) or other shaft, or coaxially surrounds it.

5. The device according to one of the preceding claims, **characterized in that** the flow connection (4a, 4b, 7, 13, 15, 35) in the region of the tube (1) or other shaft is configured as one on whose outer side a line or hose line can be attached, preferably whereby a trough-shaped groove or depression is formed in the outer side of the tube (1) or other shaft for accommodating an attachable line or hose line, especially whereby the trough-shaped groove or depression comprises lateral undercuts, so that a line or hose line, which can be pressed in or inserted there, is fixed and cannot detach spontaneously, and/or whereby the line or hose line that can be attached to the outer side of the tube (1) or of the other shaft is preformed in such a way that it fills the trough-shaped groove or depression and thereby supplements the adjacent outer contours of the tube (1) or the other shaft in a manner than maintains the contour.

6. The device according to one of the preceding claims, **characterized in that** in the transition region of the flow connection (4a, 4b, 7, 13, 15, 35) from a flow channel section (4a) formed in or integrated into the tube (1) or in another shaft to a progression of the flow channel (4b, 7, 13, 15, 35) that is detached therefrom
a) a component (17, 17a) with a ramp-shaped or arch-shaped progression is provided, in particular inserted, preferably whereby a detachable component (17, 17a) is inserted, by means of a rearward, preferably mandrel-like prolongation (17) arranged on a side facing away from the ramp (17a), into a depression aligning with the flow channel section (4a) formed in or integrated into the tube (1) or in another shaft; and/or
b) a component (18) made of a thin-walled material that nestles the outlet of the flow channel (4a) in the tube (1) or other shaft; and/or
c) a component (19) with a tubular form and a gently bent progression made of a kink-resistant material is inserted, preferably whereby the outer cross section of the component (19) with a tubular form is larger than the inner cross section of the flow channel section (4a) formed in or integrated into the tube (1) or in another shaft, preferably in such a way that it can be frictionally fixed there under local widening of the flow channel section (4a); and/or
d) a hood-shaped component (18a) with lateral, saddle-like planar extensions (18b) is attached or inserted, wherein the extensions (18b) can preferably be connected in a stabilizing manner, for example adhesively, to the tube shaft covered therewith, whereby a component (17, 17a) with a ramp-shaped or arch-shaped progression is covered by a hood-shaped component (18a), preferably whereby a component (17, 17a, 18, 18a, 19) arranged in the transition region of the flow connection (4a, 4b, 7, 13, 15, 35) from a flow channel section (4a) formed in or integrated into the tube (1) or in another shaft to a progression of the flow channel (4b, 7, 13, 15, 35) that is detached therefrom, is provided in the region of the proximal end of said component with a socket for attaching or inserting a hose.

7. The device according to one of the preceding claims, **characterized in that**
a) a filter and/or a vapor barrier (20) is preferably provided extracorporeally in the flow connection (4a, 4b, 7, 13, 15, 35), and/or
b) the inner lumen in the connector (6) preferably comprises a constant cross-sectional area over the entire length of the connector (6) in a connected state of the subcomponents thereof.

8. The device according to one of the preceding claims, **characterized in that** the pressure in a volume reservoir (26, R) of the extracorporeal regulating device (29) is set to the target pressure value for the balloon-type foil body (3).

9. The device according to one of the preceding claims, **characterized in that** an element (23) with a valve function and/or a flow-directing function is provided in the flow connection (4a, 4b, 7, 13, 15, 35), which element is preferably oriented in such a way that it opens in the case of a negative pressure in the balloon-type foil body (3) as compared to the pressure in a volume reservoir (26, R) of the extracorporeal regulating device (29) and allows a rapid volume flow into the balloon-type foil body (3), in particular even without an active regulation, preferably whereby a throttling element is connected in parallel with the element (23) with a valve function and/or a flow-directing function, in particular in such a way that an excess pressure in the balloon-type foil body (3) as compared to the pressure in a volume reservoir (26, R) of the extracorporeal regulating device (29) can gradually dissipate.

10. The device according to one of the preceding claims, **characterized in that** the pressure of a pressure source (Qi) for the filling medium in the extracorporeal regulating device (29), in particular upstream of a regulating valve, is set to a pressure value above the target value for the balloon-type foil body (3), for example to a pressure value of 100 mbar or more, preferably to a pressure value of 200 mbar or more, preferentially to a pressure value of 500 mbar or more, in particular to a pressure value of 1 bar or more, or even to a pressure value of 2 bar or more

11. The device according to one of the preceding claims, **characterized in that** a pressure sensor is arranged in the balloon-type foil body (3) so that the actual pressure value in the balloon-type foil body (3) can be detected, preferably whereby the pressure sensor in the balloon-type foil body (3) is connected or can be connected via cable to the extracorporeal regulating device (29), preferably wherein the connecting cable is laid inside the tube (1) or other shaft in a possibly additional lumen or inside the flow connection (4a).

12. The device according to one of the preceding claims, **characterized in that** the extracorporeal regulating device (29) comprises an active regulator, preferably an electronic regulator, in particular a two-point regulator, which in particular is designed in such a way that, in order to adjust the pressure inside the balloon-type foil body (3) that was detected as an actual value as constantly as possible to a predetermined or predeterminable target value, preferably whereby the two-point regulator is operated with a fixed timing frequency of for example between 100 Hz and 1000 Hz, wherein respectively a valve, for example a piezo valve, is alternatingly opened and closed between a pressure source (Qi) for the filling medium with an appropriate frequency, wherein preferably the pulsation ratio between the opening phase and the closing phase can be influenced by the regulator, in particular as a reaction to the difference between a predetermined or predeterminable target pressure value, on the one hand, and the actual pressure value measured inside the balloon-type foil body (3), on the other hand.

13. The device according to one of the preceding claims, **characterized in that** the sealing surfaces of the balloon-type foil body (3) fit closely on the wall of the respective organ or space with a sealing pressure of the balloon-type foil body (3) that acts as constantly as possible, in a sealing manner on all sides and/or in a manner that minimizes as much as possible a remaining residual space between the balloon-type foil body (3) and an adjacent structure.

14. The device according to one of the preceding claims, **characterized in that** in the case of a tracheal tube (1)
a) a defined high-volume, flow-optimized supply of the filling medium to the tracheal tube cuff (3) is provided, and/or
b) the length of a shaft-integrated supply line (4a) to the sealing balloon-type foil body (3) is reduced to a minimum, preferably such that the structural transition region from the tube (1) or shaft to the filling hose (4b) is approximately 1 to 2 cm above the level of the vocal folds (glottis), and/or
c) a turbulent flow is prevented when moving the filling medium between the reservoir or regulator (35) and the cuff (3), and/or
d) in the region of the transition from the shaft-integrated lumen (4b) in the tracheal sealing cuff (3), and/or in the region of the transition from the shaft-integrated supply lumen (4a) in the supply hose (4b) that extends extracorporeally, as well as between the connector parts (6), the flow properties are optimized to the extent that a sealing-pressure-maintaining extracorporeal volume compensation that acts in a synchronous manner can be achieved in the sealing balloon element (3), and/or
e) between connector parts (6), and/or in the region of integrated filter components or valve components (20, 23), the flow properties are optimized to the extent that a sealing-pressure-maintaining extracorporeal volume compensation that acts in a synchronous manner can be achieved in the sealing balloon element (3).

15. The device according to one of the preceding claims, **characterized in that** the sealing balloon element or the balloon-type foil body (3)
a) consist of a thin-walled balloon foil made of polyurethane, which in the segment directed towards the respective surface to be sealed has a wall thickness of 5 to 30 µm, preferably of 10 to 20 µm, and/or
b) consists of PUR with a material durometer according to Shore of 70 A to 95 A, and/or with a material durometer according to Shore of 54D to 60D, and/or
c) comprises a multilayer wall structure, wherein at least one material layer has special barrier properties for water vapor and/or air, wherein the barrier layer consists for example of EVOH

## Revendications

1. Dispositif pour l'étanchéification avec compensation de volume synchrone d'un organe d'un organe creux ou d'un espace anatomique d'une dimension anatomique prédéfinie, comprenant (i) un corps pelliculaire de type ballonnet (3) intracorporel avec des surfaces étanchéifiantes pour s'appliquer contre la paroi de l'organe creux ou espace correspondant, tandis que le corps pelliculaire de type ballonnet lui-même est rempli d'un fluide de remplissage sous une pression de consigne maximale de 50 mbar, de préférence sous une pression de consigne maximale de 40 mbar, en particulier sous une pression de consigne maximale de 30 mbar, (ii) un tube (1) ou autre tige sur lequel repose le corps pelliculaire de type ballonnet (3), (iii) un dispositif de régulation extracorporel (29) avec un réservoir volumique (26, R) et/ou une source de pression (Qi) pour le fluide de remplissage, (iv) ainsi qu'un raccordement d'écoulement (4a, 4b, 7, 13, 15, 35) entre le corps pelliculaire de type ballonnet (3) intracorporel et le dispositif de régulation extracorporel (29), qui au moins partiellement est disposé dans ou le long du tube (1) ou autre tige, un connecteur (6) étant prévu de manière extracorporelle dans le raccordement d'écoulement (4a, 4b, 7, 13, 15, 35), **caractérisé en ce que**
a) le corps pelliculaire de type ballonnet (3) est formé à une dimension résiduelle dépassant la dimension anatomique de l'organe ou de l'espace correspondant, de telle sorte que les surfaces étanchéifiantes du corps pelliculaire de type ballonnet (3) reposent au moins partiellement en formant des plis contre la paroi de l'organe creux ou de l'espace correspondant, lorsque le corps pelliculaire de type ballonnet (3) est non étiré et détendu, et **en ce que**
b) le raccordement d'écoulement (4a, 4b, 7, 13, 15, 35)
b1) entre le corps pelliculaire de type ballonnet (3) intracorporel et le dispositif de régulation extracorporel (29) dans la zone de son tracé dans ou le long du tube (1) ou autre tige, y compris une zone de transition entre le tube (1) ou autre tige et un tracé qui en est séparé exempt de déviations à angle droit, de telle sorte qu'un écoulement laminaire peut s'y former et en l'espace d'un temps de latence égal ou inférieur à 200 ms, par exemple égal ou inférieur à 100 ms, de préférence égal ou inférieur à 50 ms, en particulier égal ou inférieur à 25 ms, de telle sorte que la quantité de remplissage supplémentaire requise dans le corps pelliculaire de type ballonnet (3) peut être complétée afin de compenser des variations de la pression de remplissage du ballonnet et/ou du volume du ballonnet et/ou des pressions et forces agissant sur le corps pelliculaire de type ballonnet (3), de telle sorte que l'étanchéité ou le tamponnement par remplissage d'espace de l'organe creux ou de l'espace lors de variations dynamiques alternées de la pression de remplissage du ballonnet reste intacte en cas de chute de pression dans le corps pelliculaire de type ballonnet (3) de 30 mbar, et
b2) dans la zone de la transition entre différents composants (4a, 4b, 7, 13, 15, 35), en particulier dans la zone du connecteur (6), est sans flambage et sans bord et sans gradation et sans interstice et sans arête et sans autres protubérances ou évidements abrupts, pour ne pas gêner l'écoulement laminaire.

2. Dispositif selon la revendication 1, **caractérisé en ce que** le raccordement d'écoulement (4a, 4b, 7, 13, 15, 35) dans la zone du tube (1) ou autre tige
a) est sans flambage et/ou sans bord et/ou sans gradation et/ou sans interstice et/ou sans arête et/ou sans autres protubérances ou évidements abrupts, pour ne pas gêner l'écoulement laminaire, et/ou
b) est sans courbure, dont le rayon de courbure dans le sens longitudinal de l'écoulement est inférieur à 0,5 cm, par exemple inférieur à 1 cm, de préférence inférieur à 2 cm, en particulier inférieur à 5 cm.

3. Dispositif selon l'une des revendications précédentes, **caractérisé en ce que** la surface de section transversale du raccordement d'écoulement (4a, 4b, 7, 13, 15, 35)
a) ne diminue pas à partir de la zone du tube (1) ou de l'autre tige jusqu'au dispositif de régulation extracorporel (29), et/ou
b) augmente dans la zone de transition entre le tube (1) ou autre tige et un tracé qui en est séparé, et/ou
c) que la surface de section transversale intérieure minimale dans le raccordement d'écoulement extracorporel (4b, 7, 13, 15, 35) est supérieure à la surface de section transversale intérieure minimale de la section de canal d'écoulement intracorporelle (4a) intégrée ou moulée dans le tube (1) ou dans une autre tige, par exemple au moins 1,1 fois aussi grande que la surface de section transversale intérieure minimale de la section de canal d'écoulement intracorporelle (4a), de préférence au moins 1,2 fois aussi grande que la surface de section transversale intérieure minimale de la section de canal d'écoulement intracorporelle (4a), en particulier au moins 1,3 fois aussi grande que la surface de section transversale intérieure minimale de la section de canal d'écoulement intracorporelle (4a).

4. Dispositif selon l'une des revendications précédentes, **caractérisé en ce que** la section transversale du raccordement d'écoulement (4a, 4b, 7, 13, 15, 35) présente une forme arquée dans ou le long du tube (1) ou autre tige, qui de préférence épouse de manière approximativement tangentielle une lumière fonctionnelle à l'intérieur du tube (1) ou autre tige ou l'entoure de manière coaxiale.

5. Dispositif selon l'une des revendications précédentes, **caractérisé en ce que** le raccordement d'écoulement (4a, 4b, 7, 13, 15, 35) dans la zone du tube (1) ou autre tige est conçu sous forme de conduite ou conduite flexible pouvant être reliée à sa face extérieure, de préférence **en ce qu'**une goulotte en forme de cuvette ou cavité est formée dans la face extérieure du tube (1) ou autre tige pour la réception d'une conduite ou conduite flexible pouvant être ajoutée, en particulier **en ce que** la goulotte en forme de cuvette ou cavité présente des contre-dépouilles latérales, de telle sorte qu'une conduite ou conduite flexible qui peut y être enfoncée ou enfichée est retenue et ne peut pas se détacher automatiquement, et/ou **en ce que** le cas échéant la conduite ou conduite flexible pouvant être ajoutée sur la face extérieure du tube (1) ou autre tige est préformée de telle sorte qu'elle remplit la goulotte en forme de cuvette ou cavité en complétant, tout en maintenant les contours, les contours extérieurs adjacents du tube (1) ou autre tige.

6. Dispositif selon l'une des revendications précédentes, **caractérisé en ce que** dans la zone de transition du raccordement d'écoulement (4a, 4b, 7, 13, 15, 35) entre une section de canal d'écoulement (4a) intégrée ou moulée dans le tube (1) ou dans une autre tige et un tracé du canal d'écoulement (4b, 7, 13, 15, 35) qui en est séparé
a) est prévu, en particulier est intégré, un composant (17, 17a) en forme de rampe ou d'arc, de préférence **en ce qu'**un composant désolidarisable (17, 17a) est inséré dans un évidement aligné avec la section de canal d'écoulement (4a) intégrée ou moulée dans le tube (1) ou dans une autre tige au moyen d'un prolongement (17), de préférence en forme de dôme, disposé à l'arrière, sur un côté opposé à la rampe (17a), et/ou
b) est inséré un composant (18) constitué d'un matériau à paroi mince, s'adaptant à l'ouverture du canal d'écoulement (4a) dans le tube (1) ou autre tige, et/ou
c) est inséré un composant (19) de forme tubulaire et légèrement recourbé constitué d'un matériau résistant au flambage, de préférence **en ce que** la section transversale extérieure du composant (19) de forme tubulaire est supérieure à la section transversale intérieure de la section de canal d'écoulement (4a) intégrée ou moulée dans le tube (1) ou dans une autre tige, de préférence de telle sorte qu'elle peut y être fixée par friction sous étirement local de la section de canal d'écoulement (4a), et/ou
d) est ajouté ou intégré un composant (18a) en forme de cloche avec des ramifications (18b) latérales planes en forme de selle, **en ce que** les ramifications (18b) peuvent être reliées de manière stabilisante de préférence à la tige de tube qui en est recouverte, par exemple par adhérence, **en ce que** le cas échéant un composant (17, 17a) présentant un tracé en forme de rampe ou d'arc est recouvert par un composant en forme de cloche (18a), de préférence **en ce qu'**un composant (17, 17a, 18, 18a, 19) disposé dans la zone de transition du raccordement d'écoulement (4a, 4b, 7, 13, 15, 35) entre une section de canal d'écoulement (4a) intégrée ou moulée dans le tube (1) ou dans une autre tige et un tracé du canal d'écoulement (4b, 7, 13, 15, 35) qui en est séparé est prévu avec un manchon pour la fixation ou introduction d'un tuyau flexible dans la zone de son extrémité proximale.

7. Dispositif selon l'une des revendications précédentes, **caractérisé**
a) **en ce qu'**un filtre et/ou une barrière de vapeur (20) de préférence de manière extracorporelle est prévu dans le raccordement d'écoulement (4a, 4b, 7, 13, 15, 35), et/ou
b) **en ce que** la lumière intérieure dans le connecteur (6) présente une surface de section transversale constante sur toute la longueur du connecteur (6) à l'état connecté de ses composants partiels.

8. Dispositif selon l'une des revendications précédentes, **caractérisé en ce que** la pression dans un réservoir volumique (26, R) du dispositif de régulation extracorporel (29) est réglée sur la valeur de consigne de pression pour le corps pelliculaire de type ballonnet (3).

9. Dispositif selon l'une des revendications précédentes, **caractérisé en ce que** dans le raccordement d'écoulement (4a, 4b, 7, 13, 15, 35) est prévu un élément (23) doté d'une fonction de vanne et/ou d'une fonction à direction de flux, ledit élément étant orienté de préférence de telle sorte qu'il s'ouvre en cas de sous-pression dans le corps pelliculaire de type ballonnet (3) par rapport à la pression dans un réservoir volumique (26, R) du dispositif de régulation extracorporel (29) et permet un flux volumétrique rapide dans le corps pelliculaire de type ballonnet (3), en particulier aussi sans régulation active, de préférence **en ce qu'**un élément d'étranglement est monté en parallèle par rapport à l'élément (23) doté d'une fonction de vanne et/ou d'une fonction à direction de flux, en particulier de telle sorte qu'une sous-pression dans le corps pelliculaire de type ballonnet (3) peut se réduire progressivement par rapport à la pression dans un réservoir volumique (26, R) du dispositif de régulation extracorporel (29).

10. Dispositif selon l'une des revendications précédentes, **caractérisé en ce que** la pression d'une source de pression (Qi) pour le fluide de remplissage dans le dispositif de régulation extracorporel (29), en particulier en amont d'une vanne de régulation, est réglée sur une valeur de pression au-dessus de la valeur de consigne pour le corps pelliculaire de type ballonnet (3), par exemple sur une valeur de pression égale ou supérieure à 100 mbar, de préférence sur une valeur de pression égale ou supérieure à 200 mbar, de préférence sur une valeur de pression égale ou supérieure à 500 mbar, en particulier sur une valeur de pression égale ou supérieure à 1 bar, voire sur une valeur de pression égale ou supérieure à 2 bar.

11. Dispositif selon l'une des revendications précédentes, **caractérisé en ce qu'**un capteur de pression est disposé dans le corps pelliculaire de type ballonnet (3), de telle sorte que la valeur réelle de pression dans le corps pelliculaire de type ballonnet (3) peut être détectée, de préférence **en ce que** le capteur de pression dans le corps pelliculaire de type ballonnet (3) est relié ou peut être relié par câble au dispositif de régulation extracorporel (29), de préférence **en ce que** le câble de raccordement à l'intérieur du tube (1) ou autre tige est posé le cas échéant dans une lumière additionnelle ou dans le raccordement d'écoulement (4a).

12. Dispositif selon l'une des revendications précédentes, **caractérisé en ce que** le dispositif de régulation extracorporel (29) comporte un régulateur actif, de préférence un régulateur électronique, en particulier un régulateur à deux positions qui est en particulier conçu pour régler de manière la plus constante possible la pression détectée comme valeur réelle à l'intérieur du corps pelliculaire de type ballonnet (3) sur une valeur de consigne prédéfinie ou qui peut être prédéfinie, de préférence **en ce que** le régulateur à deux positions fonctionne à une fréquence élémentaire fixe comprise par exemple entre 100 Hz et 1000 Hz, **en ce que** respectivement une vanne, par exemple une vanne piézoélectrique, entre une source de pression (Qi) pour le fluide de remplissage est alternativement ouverte et fermée à une fréquence correspondante, **en ce que** de préférence le rapport d'impulsions entre la phase d'ouverture et de fermeture peut être influencé par le régulateur, en particulier comme réaction à la différence entre une valeur de consigne de pression prédéfinie ou qui peut être prédéfinie d'une part et la valeur réelle de pression mesurée à l'intérieur du corps pelliculaire de type ballonnet (3) d'autre part.

13. Dispositif selon l'une des revendications précédentes, **caractérisé en ce que** les surfaces étanchéifiantes du corps pelliculaire de type ballonnet (3) étanchéifient la paroi de l'organe ou de l'espace correspondant de tous les côtés avec une pression d'étanchéité ayant une action la plus constante possible du corps pelliculaire de type ballonnet (3) et/ou reposent de manière à minimiser le plus possible un espace résiduel éventuel entre le corps pelliculaire de type ballonnet (3) et une structure adjacente.

14. Dispositif selon l'une des revendications précédentes, **caractérisé en ce que** pour un tube trachéal (1)
a) est prévu un apport défini à flux optimisé en fluide de remplissage par l'intermédiaire d'une lumière de grand diamètre vers le manchon du tube trachéal (3), et/ou
b) la longueur entre une conduite d'alimentation intégrée à la tige (4a) et le corps pelliculaire de type ballonnet (3) à étanchéifier est réduite à un minimum, de préférence de telle sorte que la zone de transition structurale du tube (1) ou tige au tuyau flexible de remplissage (4b) est approximativement de 1 à 2 cm au-dessus du plan de cordes vocales (glotte), et/ou
c) un flux turbulent lors du déplacement du fluide de remplissage entre le réservoir ou régulateur (35) et le manchon (3) est évité, et/ou
d) dans la zone de la transition de la lumière intégrée à la tige (4b) dans le manchon (3) à étanchéification trachéale, et/ou dans la zone de la transition de la lumière de conduite d'alimentation (4a) intégrée à la tige dans le tuyau flexible d'alimentation (4b) s'étendant de manière extracorporelle, ainsi qu'entre les parties du connecteur (6), les caractéristiques de flux sont optimisées de telle sorte qu'une compensation de volume extracorporelle, maintenant la pression d'étanchéité dans l'élément de ballonnet (3) étanchéifiant, peut être obtenue de manière synchrone, et/ou
e) entre les parties du connecteur (6), et/ou dans la zone de composants filtre ou vanne (20, 23) intégrés, les caractéristiques de flux sont optimisées de telle sorte qu'une compensation de volume extracorporelle, maintenant la pression d'étanchéité dans l'élément de ballonnet (3) étanchéifiant, peut être obtenue de manière synchrone.

15. Dispositif selon l'une des revendications précédentes, **caractérisé en ce que** l'élément de ballonnet étanchéifiant ou le corps pelliculaire de type ballonnet (3)
a) est constitué d'un film de ballonnet à paroi mince en polyuréthane, qui présente dans le segment orienté vers la surface respective à étanchéifier une épaisseur de paroi de 5 à 30 µm, de préférence de 10 à 20 µm, et/ou
b) est constitué de PUR d'une dureté Shore de 70 A à 95 A, et/ou d'une dureté Shore de 54 D à 60 D, et/ou
c) présente une structure de paroi multicouche, **en ce qu'**au moins une couche de matériau présente des propriétés barrière particulières pour la vapeur d'eau et/ou l'air, la couche barrière étant par exemple constituée de EVOH.
